# EUROPEAN PATENT SPECIFICATION

(11) **EP 1 257 818 B1**
(45) Date of publication and mention of the grant of the patent: **05.01.2011**
(21) Application number: 00961466.0
(22) Date of filing: 01.09.2000
(51) Int. Cl.: G01N 33/566

(54) **ENZYME-BASED G-PROTEIN-COUPLED RECEPTOR ASSAY**
AUF ENZYMEN BASIERENDER ASSAY FÜR G-PROTEIN-GEKOPPELTE REZEPTOREN
PROCÉDÉ D'ANALYSE ENZYMATIQUE DE RECEPTEURS COUPLÉS AUX PROTEINES G

(30) Priority: 07.02.2000 US 180669 P
(43) Date of publication of application: 20.11.2002
(73) Proprietor: Life Technologies Corporation, Carlsbad, CA 92008 (US)
(72) Inventor: PALMER, Michelle, A., J., Arlington, MA 02174 (US); GEE, Melissa, Grafton, MA 01519 (US); TILLOTSON, Bonnie, Belmont, MA 02178 (US); CHANG, Xia-Jia, Lincoln, MA 01773 (US)
(74) Representative: Ruffles, Graham Keith
(86) International application number: PCT/US2000/024043
(87) International publication number: WO 2001/059451

(56) References cited:
- WO-A-98/44350
- US-A- 5 891 646
- US-A- 5 891 646
- BT. BLAKELY ET AL.: "Epidermal growth factor receptor dimerization monitored in live cells." NATURE BIOTECHNOLOGY, vol. 18, 1 February 2000 (2000-02-01), pages 218-222, XP002338962
- JORDAN BRYEN A ET AL: "G-protein-coupled receptor heterodimerization modulates receptor function" NATURE, MACMILLAN JOURNALS LTD. LONDON, GB, vol. 399, no. 6737, 17 June 1999 (1999-06-17), pages 697-700, XP002306507 ISSN: 0028-0836
- BERTIN B ET AL: "Cellular signaling by agonist-activated receptor/Gsalpha fusion protein" PROCEEDINGS OF THE NATIONAL ACADEMY OF SCIENCES OF USA, NATIONAL ACADEMY OF SCIENCE. WASHINGTON, US, vol. 91, no. 19, 13 September 1994 (1994-09-13), pages 8827-8831, XP002114989 ISSN: 0027-8424
- BARAK ET AL.: 'A beta-arrestin/green fluorescent protein biosensor for detecting G protein-coupled receptor activation' THE JOURNAL OF BIOLOGICAL CHEMISTRY vol. 272, no. 44, 31 October 1997, pages 27497 - 27500, XP002934155
- ROSSI ET AL.: 'Monitoring protein-protein interactions in intact eukaryotic cells by beta-galactosidase complementation' PROC. NATL. ACAD. SCI. USA vol. 94, August 1998, pages 8405 - 8410, XP002934156

## Description

### BACKGROUND OF THE INVENTION

This application claims the benefit from Provisional Application Serial No. 60/180,669, filed February 7, 2000.

### Field of the Invention

This invention relates to methods of detecting G-protein-coupled receptor (GPCR) activity, and provides methods of assaying GPCR activity and methods for screening for GPCR ligands, G-protein-coupled receptor kinase (GRK) activity, and compounds that interact with components of the GPCR regulatory process.

The actions of many extracellular signals are mediated by the interaction of G-protein-coupled receptors (GPCRs) and guanine nucleotide-binding regulatory proteins (G-proteins). G-protein-mediated signaling systems have been identified in many divergent organisms, such as mammals and yeast. The GPCRs represent a large super family of proteins which have divergent amino acid sequences, but share common structural features, in particular, the presence of seven transmembrane helical domains. GPCRs respond to, among other extracellular signals, neurotransmitters, hormones, odorants and light. Individual GPCR types activate a particular signal transduction pathway; at least ten different signal transduction pathways are known to be activated via GPCRs. For example, the beta 2-adrenergic receptor (β2AR) is a prototype mammalian GPCR. In response to agonist binding, β2AR receptors activate a G-protein (Gs) which in turn stimulates adenylate cyclase activity and results in increased cyclic adenosine monophosphate (cAMP) production in the cell.

The signaling pathway and final cellular response that result from GPCR stimulation depends on the specific class of G-protein with which the particular receptor is coupled (Hamm, "The many faces of G-Protein Signaling." J. Biol. Chem., 273:669-672 (1998)). For instance, coupling to the Gs class of G-proteins stimulates cAMP production and activation of Protein Kinase A and C pathways, whereas coupling to the Gi class of G-proteins down regulates cAMP. Other second messenger systems as calcium, phosphlipase C, and phosphatidylinositol 3 may also be utilized. As a consequence, GPCR signaling events have predominantly been measured via quantification of these second messenger products.

A common feature of GPCR physiology is desensitization and recycling of the receptor through the processes of receptor phosphorylation, endocytosis and dephosphorylation (Ferguson, et al., "G-protein-coupled receptor regulation: role of G-protein-coupled receptor kinases and arrestins." Can. J. Physiol. Pharmacol., 74:1095-1110 (1996)). Ligand-occupied GPCRs can be phosphorylated by two families of serine/threonine kinases, the G-protein-coupled receptor kinases (GRKs) and the second messenger-dependent protein kinases such as protein kinase A and protein kinase C. Phosphorylation by either class of kinases serves to down-regulate the receptor by uncoupling it from its corresponding G-protein. GRK-phosphorylation also serves to down-regulate the receptor by recruitment of a class of proteins known as the arrestins that bind the cytoplasmic domain of the receptor and promote clustering of the receptor into endocytic vescicles. Once the receptor is endocytosed, it will either be degraded in lysosomes or dephosphorylated and recycled back to the plasma membrane as fully-functional receptor.

Binding of an arrestin protein to an activated receptor has been documented as a common phenomenon for a variety of GPCRs ranging from rhodopsin to β2AR to the neurotensin receptor (Barak, et al., "A (β-arrestin/Green Fluorescent fusion protein biosensor for detecting G-Protein-Coupled Receptor Activation," J. Biol. Chem., 272:27497-500 (1997)). Consequently, monitoring arrestin interaction with a specific GPCR can be utilized as a generic tool for measuring GPCR activation. Similarly, a single G-protein and GRK also partner with a variety of receptors (Hamm, et al. (1998) and Pitcher et al., "G-Protein-Coupled Receptor Kinases," Annu. Rev. Biochem., 67:653-92 (1998)), such that these protein/protein interactions may also be monitored to determine receptor activity.

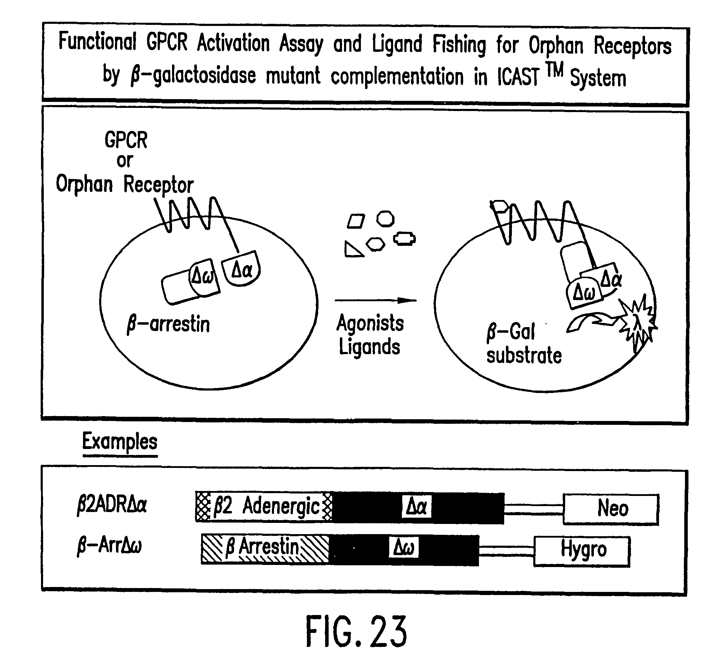

The present invention involves the use of a proprietary technology (ICAST^{™}, Intercistronic Complementation Analysis Screening Technology) for monitoring protein/protein interactions in GPCR signaling. The method involves using two inactive β-galactosidase mutants, each of which is fused with one of two interacting protein pairs, such as a GPCR and an arrestin. The formation of an active (β-galactosidase complex is driven by interaction of the target proteins. In this system, β-galactosidase activity acts as a read out of GPCR activity. FIGURE 23 is a schematic depicting the method of the present application. FIGURE 23 shows two inactive mutants that become active when they interact. In addition, this technology could be used to monitor GPCR-mediated signaling pathways via other downstream signaling components such as G-proteins, GRKs or c-Src.

Many therapeutic drugs in use today target GPCRs, as they regulate vital physiological responses, including vasodilation, heart rate, bronchodilation, endocrine secretion and gut peristalsis. See, e.g., Lefkowitz et al., Annu. Rev. Biochem., 52:159 (1983). For instance, drugs targeting the highly studied GPCR, β2AR are used in the treatment of anaphylaxis, shock hypertension, asthma and other conditions. Some of these drugs mimic the ligand for this receptor. Other drugs act to antagonize the receptor in cases when disease arises from spontaneous activity of the receptor.

Efforts such as the Human Genome Project are identifying new GPCRs ("orphan" receptors) whose physiological roles and ligands are unknown. It is estimated that several thousand GPCRs exist in the human genome. Of the 250 GPCRs identified to date, only 150 have been associated with ligands.

### SUMMARY OF THE INVENTION

The invention is set forth in the appended claims which alone define its scope.

A first aspect of the present application is a method that monitors GPCR function proximally at the site of receptor activation, thus providing more information for drug discovery purposes due to fewer competing mechanisms. Activation of the GPCR is measured by a read-out for interaction of the receptor with a regulatory component such as arrestin, G-protein, GRK or other kinases, the binding of which to the receptor is dependent upon agonist occupation of the receptor. Protein/protein interaction is detected by complementation of reporter proteins such as utilized by the ICAST^{™} technology.

A further aspect of the present application is a method of assessing G-protein-coupled receptor (GPCR) pathway activity under test conditions by providing a test cell that expresses a GPCR, e.g., muscarinic, adrenergic, dopamine, angiotensin or endothelin, as a fusion protein to a mutant reporter protein and interacting, i.e., G-proteins, arrestin or GRK, as a fusion protein with a complementing reporter protein. When test cells are exposed to a known agonist to the target GPCR under test conditions, activation of the GPCR will be monitored by complementation of the reporter enzyme. Increased reporter enzyme activity reflects interaction of the GPCR with its interacting protein partner.

A further aspect of the present application is a method of assessing GPCR pathway activity in the presence of a test kinase.

A further aspect of the present application is a method of assessing GPCR pathway activity in the presence of a test G-protein.

A further aspect of the present application is a method of assessing GPCR pathway activity upon exposure of the test cell to a test ligand.

A further aspect of the present application is a method of assessing GPCR pathway activity upon co-expression in the test cell of a second receptor.

A further aspect of the present application is a method for screening for a ligand or agonists to an orphan GPCR. The ligand or agonist could be contained in natural or synthetic libraries or mixtures or could be a physical stimulus. A test cell is provided that expresses the orphan GPCR as a fusion protein with one β-galactosidase mutant and, for example, an arrestin or mutant form of arrestin as a fusion protein with another β-galactosidase mutant. The interaction of the arrestin with the orphan GPCR upon receptor activation is measured by enzymatic activity of the complemented β-galactosidase. The test cell is exposed to a test compound, and an increase in β-galactosidase activity indicates the presence of a ligand or agonist.

A further aspect of the present application is a method for screening a protein of interest, for example, an arrestin protein (or mutant form of the arrestin protein) for the ability to bind to a phosphorylated, or activated, GPCR. A cell is provided that expresses a GPCR and contains β-arrestin. The cell is exposed to a known GPCR agonist and then reporter enzyme activity is detected. Increased reporter enzyme activity indicates that the β-arrestin molecule can bind to phosphorylated, or activated, GPCR in the test cell.

A further aspect of the present application is a method to screen for an agonist to a specific GPCR. The agonist could be contained in natural or synthetic libraries or could be a physical stimulus. A test cell is provided that expresses a GPCR as a fusion protein with one β-galactosidase mutant and, for example, an arrestin as a fusion protein with another β-galactosidase mutant. The interaction of arrestin with the GPCR upon receptor activation is measured by enzymatic activity of the complemented β-galactosidase. The test cell is exposed to a test compound, and an increase in β-galactosidase activity indicates the presence of an agonist. The test cell may express a known GPCR or a variety of known GPCRs, or may express an unknown GPCR or a variety of unknown GPCRs. The GPCR may be, for example, an odorant GPCR or a βAR GPCR.

A further aspect of the present application is a method of screening a test compound for G-protein-coupled receptor (GPCR) antagonist activity. A test cell is provided that expresses a GPCR as a fusion protein with one β-galactosidase mutant and, for example, an arrestin as a fusion protein with another β-galactosidase mutant. The interaction of arrestin with the GPCR upon receptor activation is measured by enzymatic activity of the complemented β-galactosidase. The test cell is exposed to a test compound, and an increase in β-galactosidase activity indicates the presence of an agonist. The cell is exposed to a test compound and to a GPCR agonist, and reporter enzyme activity is detected. When exposure to the agonist occurs at the same time as or subsequent to exposure to the test compound, a decrease in β-galactosidase activity after exposure to the test compound indicates that the test compound has antagonist activity to the GPCR.

A further aspect of the present application is a method of screening a sample solution for the presence of an agonist, antagonist or ligand to a G-protein-coupled receptor (GPCR). A test cell is provided that expresses a GPCR fusion and contains, for example, a β-arrestin protein fusion. The test cell is exposed to a sample solution, and reporter enzyme activity is assessed. Changed reporter enzyme activity after exposure to the sample solution indicates the sample solution contains an agonist, antagonist or ligand for a GPCR expressed in the cell.

A further aspect of the present application is a method of screening a cell for the presence of a G-protein-coupled receptor (GPCR).

A further aspect of the present application is a method of screening a plurality of cells for those cells which contain a G-protein coupled receptor (GPCR).

A further aspect of the application is a method for mapping GPCR-mediated signaling pathways. For instance, the system could be utilized to monitor interaction of c-src with β-arrestin-1 upon GPCR activation. Additionally, the system could be used to monitor protein/protein interactions involved in cross-talk between GPCR signaling pathways and other pathways such as that of the receptor tyrosine kinases or Ras/Raf.

A further aspect of the application is a method for monitoring homo- or hetero-dimerization of GPCRs upon agonist or antagonist stimulation.

A further aspect of the application is a method of screening a cell for the presence of a G-protein-coupled receptor (GPCR) responsive to a GPCR agonist. A cell is provided that contains protein partners that interact downstream in the GPCR's pathway. The protein partners are expressed as fusion proteins to the mutant, complementing enzyme and are used to monitor activation of the GPCR. The cell is exposed to a GPCR agonist and then enzymatic activity of the reporter enzyme is detected. Increased reporter enzyme activity indicates that the cell contains a GPCR responsive to the agonist.

The application is achieved by using ICAST^{™} protein/protein interaction screening to map signaling pathways. This technology is applicable to a variety of known and unknown GPCRs with diverse functions. They include, but are not limited to, the following subfamilies of GPCRs:
(a) receptors that bind to amine-like ligands-Acetylcholine muscarinic receptor (M1 to M5), alpha and beta Adrenoceptors, Dopamine.receptors (D1, D2, D3 and D4), Histamine receptors (H1 and H2), Octopamine receptor and Serotonin receptors (5HT1, 5HT2, 5HT4, 5HT5, 5HT6, 5HT7);
(b) receptors that bind to a peptide ligand-Angiotensin receptor, Bombesin receptor, Bradykinin receptor, C-C chemokine receptors (CCR1 to CCR8, and CCR10), C-X-C type Chemokine receptors (CXC-R5), Cholecystokinin type A receptor, CCK type receptors, Endothelin receptor, Neurotesin receptor, FMLP-related receptors, Somatostatin receptors (type 1 to type 5) and Opioid receptors (type D, K, M, X);
(c) receptors that bind to hormone proteins- Follic stimulating hormone receptor, Thyrotrophin receptor and Lutropin-choriogonadotropic hormone receptor;
(d) receptors that bind to neurotransmitters-substance P receptor, Substance K receptor and neuropeptide Y receptor;
(e) Olfactory receptors-Olfactory type 1 to type 11, Gustatory and odorant receptors;
(f) Prostanoid receptors-Prostaglandin E2 (EP1 to EP4 subtypes), Prostacyclin and Thromboxane;
(g) receptors that bind to metabotropic substances-Metabotropic glutamate group I to group III receptors;
(h) receptors that respond to physical stimuli, such as light, or to chemical stimuli, such as taste and smell; and
(i) orphan GPCRs-the natural ligand to the receptor is undefined.

ICAST^{™} provides many benefits to the screening process, including the ability to monitor protein interactions in any sub-cellular compartment-membrane, cytosol and nucleus; the ability to achieve a more physiologically relevant model without requiring protein overexpression; and the ability to achieve a functional assay for receptor binding allowing high information content.

### BRIEF DESCRIPTION OF THE DRAWINGS

FIGURE 1. Cellular expression levels of β2 adrenergic receptor (β2AR) and β-arrestin-2 (βArr2) in C2 clones. Quantification of β-gal fusion protein was performed using antibodies against β-gal and purified β-gal protein in a titration curve by a standardized ELISA assay. Figure 1A shows expression levels of β2AR-βgalΔα clones (in expression vector pICAST ALC). Figure 1B shows expression levels of βArr2-βgalΔω in expression vector pICAST OMC4 for clones 9-3, -7, -9, -10, -19 and -24, or in expression vector pICAST OMN4 for clones 12-4, -9, -16, -18, -22 and -24.

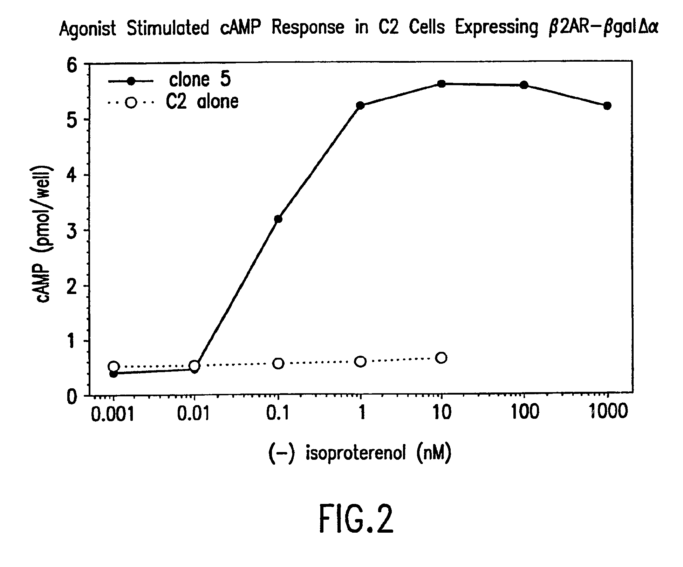

FIGURE 2. Receptor β2AR activation was measured by agonist-stimulated cAMP production. C2 cells expressing pICAST ALC β2AR (clone 5) or parental cells were treated with increasing concentrations of (-)isoproterenol and 0.1 mM IBMX. The quantification of cAMP level was expressed as pmol/well.

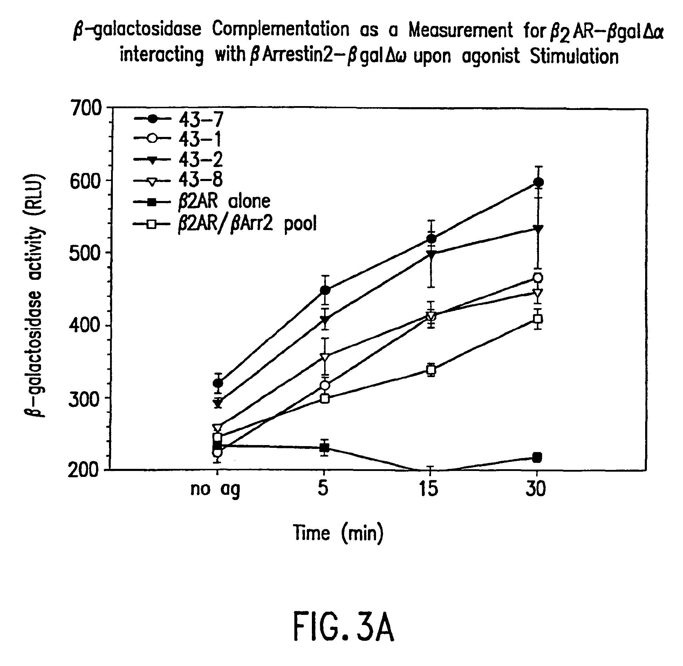

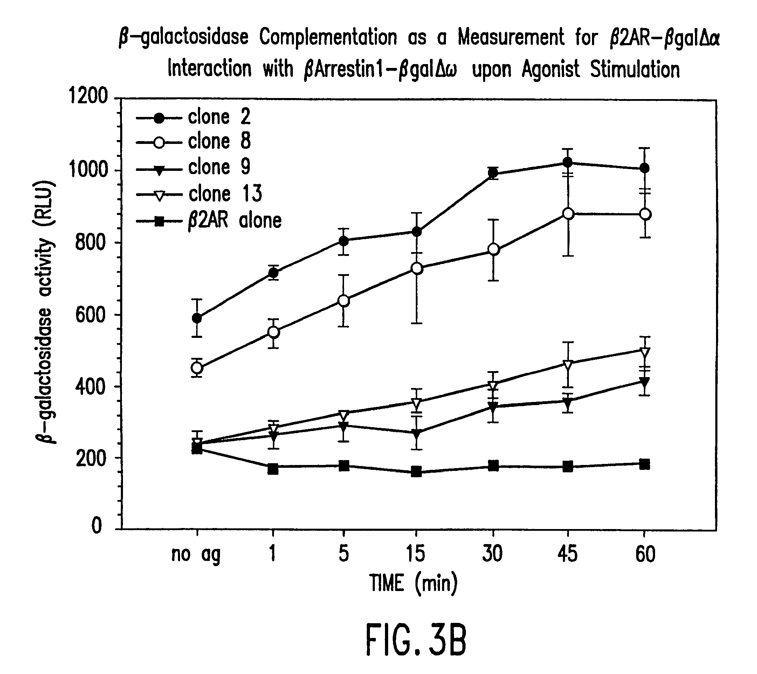

FIGURE 3. Interaction of activated receptor β2AR and arrestin can be measured by β-galactosidase complementation. Figure 3A shows a time course of β-galactosidase activity in response to agonist (-)isoproterenol stimulation in C2 expressing β2AR-βgalΔα (β2AR alone, in expression vector pICAST ALC), or C2 clones, and a pool of C2 co-expressing β32AR-βgalΔα and βArr2-βgalΔω (in expression vectors pICAST ALC and pICAST OMC). Figure 3B shows a time course of β galactosidase activity in response to agonist (-)isoproterenol stimulation in C2 cells expressing β2AR alone (in expression vector pICAST ALC) and C2 clones co-expressing β2AR and βArr1 (in expression vectors ICAST ALC and pICAST OMC).
FIGURE 4. Agonist dose response for interaction of β2AR and arrestin can be measured by β-galactosidase complementation. Figure 4A shows a dose response to agonists (-)isoproterenol and procaterol in C2 cells co-expressing pICAST ALC β2AR and pICAST OMC βArr2 fusion constructs. Figure 4B shows a dose response to agonists (-)isoproterenol and procaterol in C2 cells co-expressing pICAST ALC β2AR and pICAST OMC βArr1 fusion constructs.

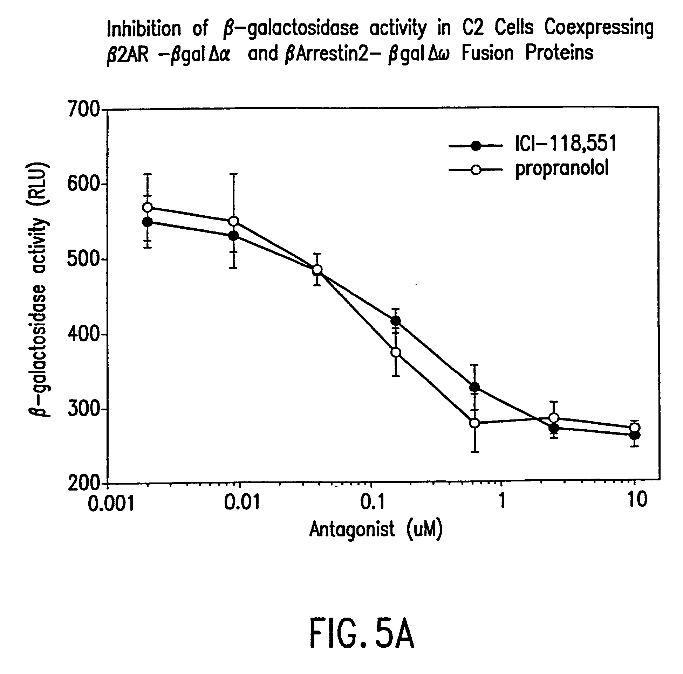

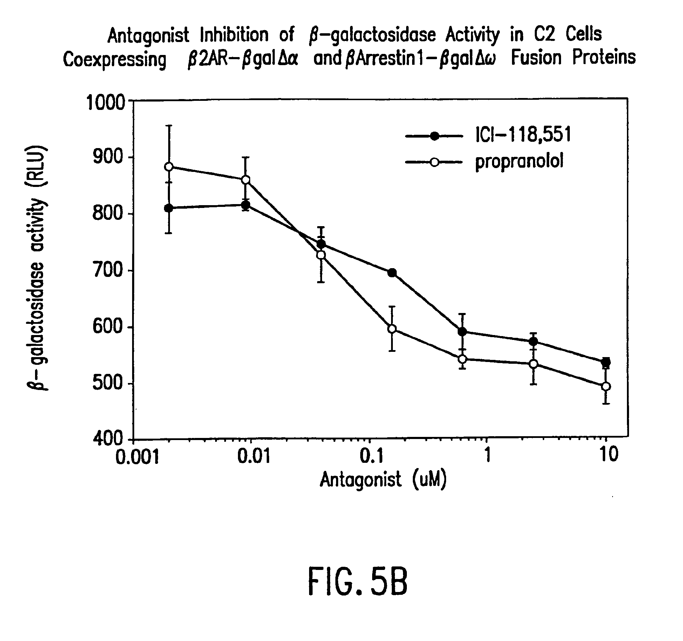

FIGURE 5. Antagonist mediated inhibition of receptor activity can be measured by β-galactosidase complementation in cells co-expressing β2AR-βgalΔα and βArr-βga1Δω. Figure 5A shows specific inhibition with adrenergic antagonists ICI-118,551 and propranolol of β-galactosidase activity in C2 clones co-expressing pICAST ALC β2AR and pICAST OMC βArr2 fusion constructs after incubation with agonist (-)isoproterenol. Figure 5B shows specific inhibition of β-galactosidase activity with adrenergic antagonists ICI-118,551 and propranolol in C2 clones co-expressing pICAST ALC β2AR and pICAST OMC βArry1 fusion constructs in the presence of agonist (-)isoproterenol.

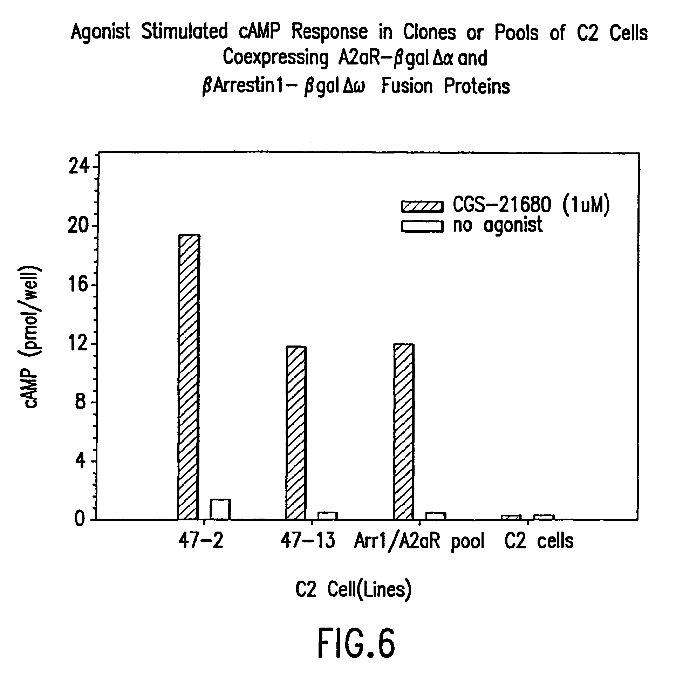

FIGURE 6. C2 cells expressing adenosine receptor A2a show cAMP induction in response to agonist (CGC-21680) treatment. C2 parental cells and C2 cells co-expressing pICAST ALC A2aR and pICAST OMC βArr1 as a pool or as selected clones were measured for agonist-induced cAMP response (pmol/well).

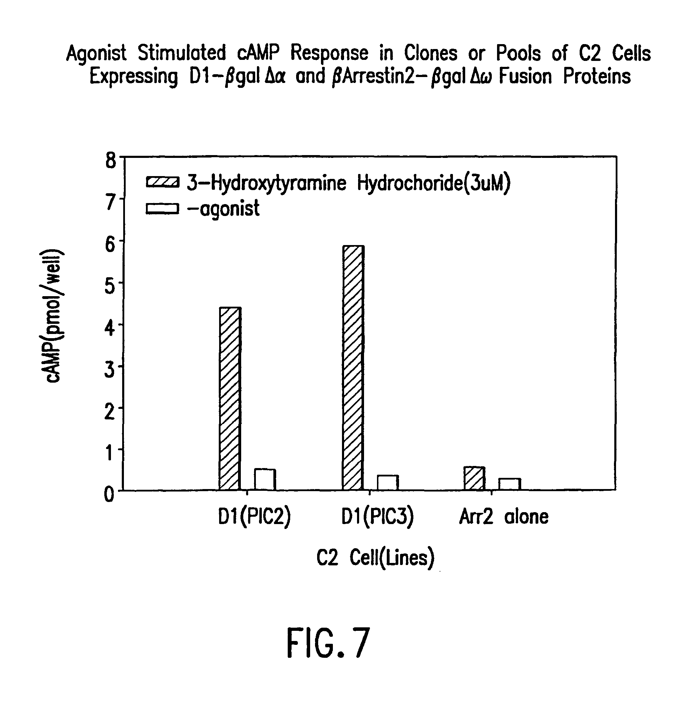

FIGURE 7. Agonist stimulated cAMP response in C2 cells co-expressing Dopamine receptorD1 (D1-βgalΔα) and β-arrestin-2 (βArr2-βgalΔω). The clone expressing βArr2-βgalΔω (Arr2 alone) was used as a negative control in the assay. Cells expressing D1-βgalΔα in addition to βArr2-βgalΔω responded agonist treatment (3-hydroxytyramine hydrochloride at 3 µM). D1(PIC2) or D1(PIC3) designate D1 in expression vector pICAST ALC2 or pICAST ALC4, respectively.

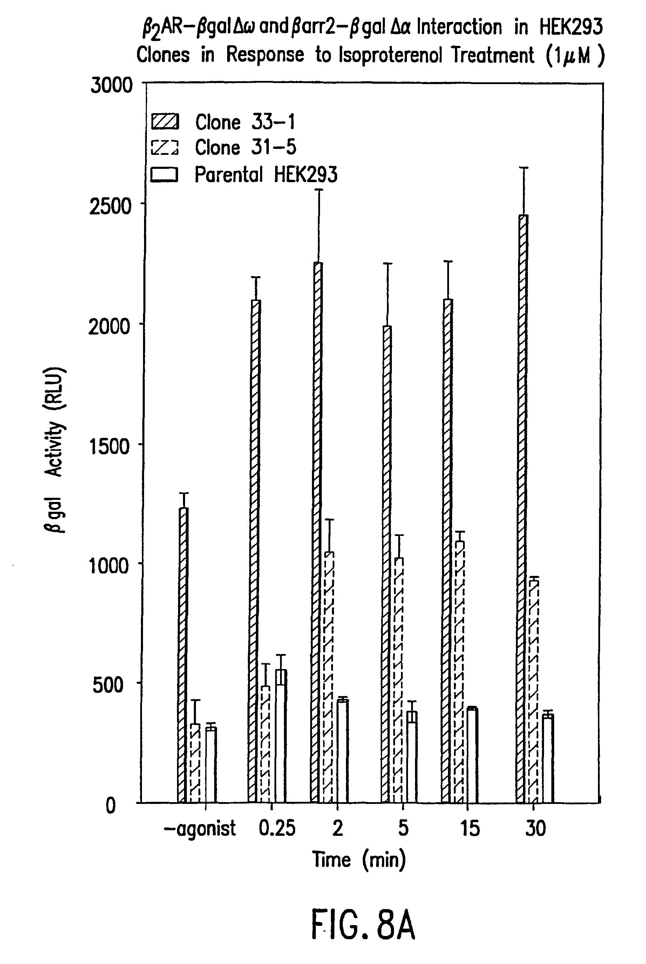

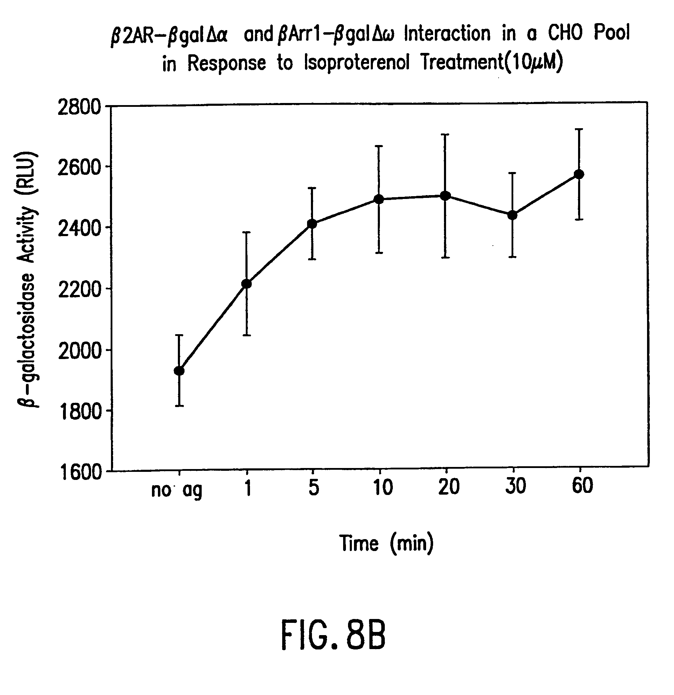

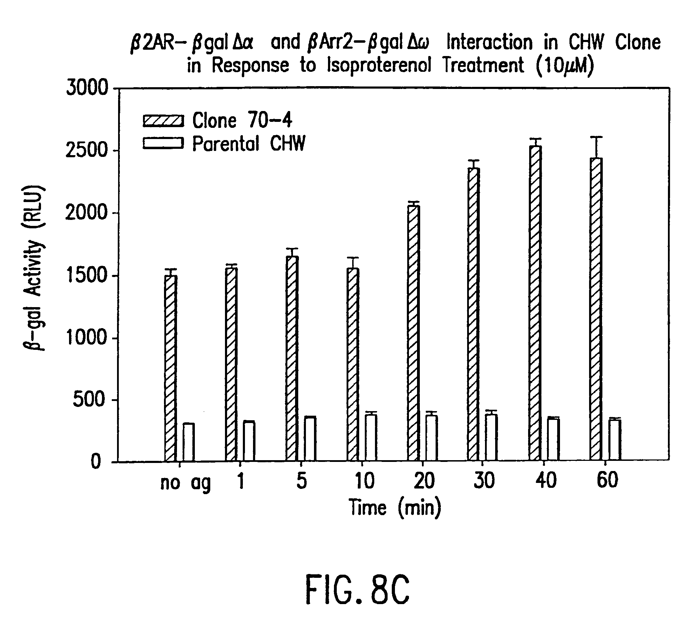

FIGURE 8. Variety of mammalian cell lines can be used to generate stable cells for monitoring GPCR and arrestin interactions. FIGURE 8A, FIGURE 8B and FIGURE 8C show the examples of HEK293, CHO and CHW cell lines co-expressing adrenergic receptor β2AR and arrestin fusion proteins of β-galactosidase mutants. The β-galactosidase activity was used to monitor agonist-induced interaction of β2AR and arrestin proteins.

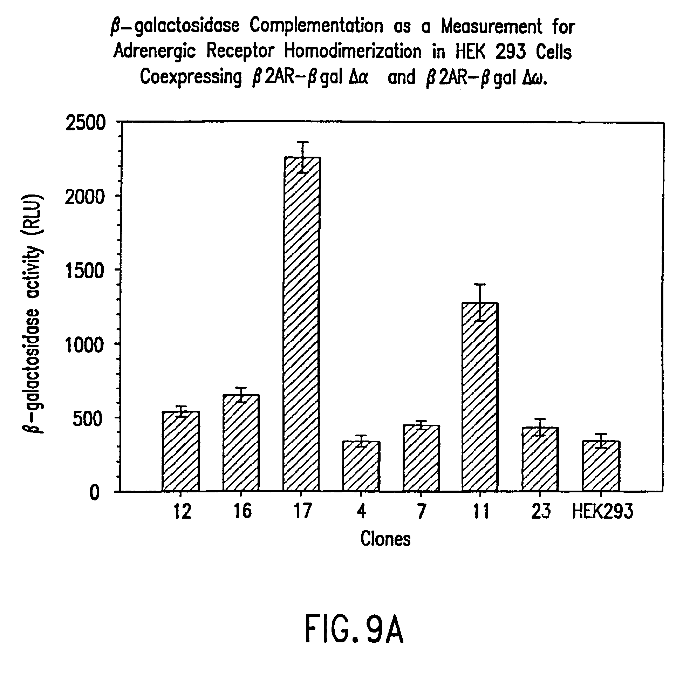

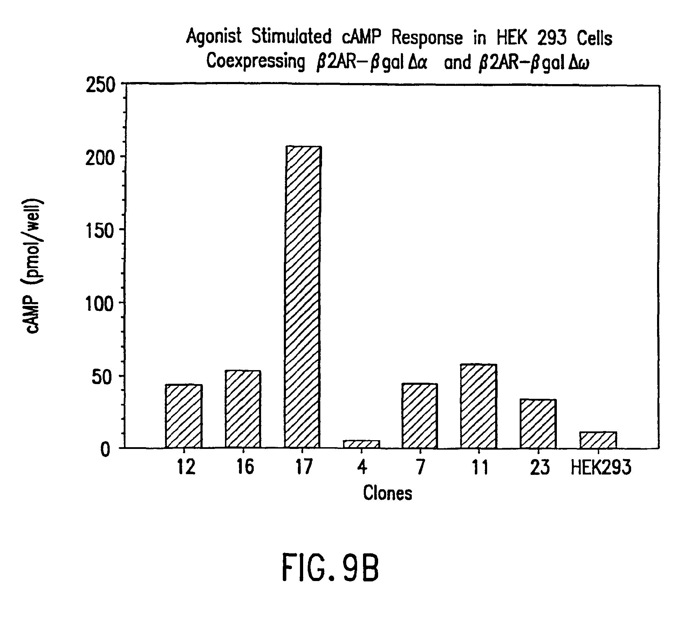

FIGURE 9. Beta-gal complementation can be used to monitor β2 adrenergic receptor homo-dimerization. FIGURE 9A shows β-galactosidase activity in HEK293 clones co-expressing pICAST ALC β2AR and pICAST OMC β2AR. FIGURE 9B shows a cAMP response to agonist (-)isoproterenol in HEK 293 clones co-expressing pICAST ALC β2AR and pICAST OMC β2AR. HEK293 parental cells were included in the assays as negative controls.

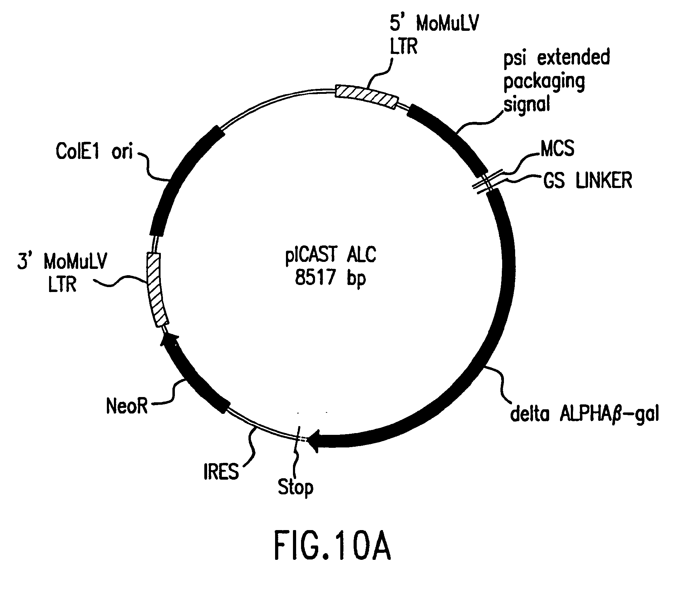

FIGURE 10A. pICAST ALC: Vector for expression of β-galΔα as a C-terminal fusion to the target protein. This construct contains the following features: MCS, multiple cloning site for cloning the target protein in frame with the β-galΔα; GS Linker, (GGGGS)n; NeoR, neomycin resistance gene; IRES, internal ribosome entry site; ColE1ori, origin of replication for growth in E. coli; 5'MoMuLV LTR and 3'MoMuLV LTR, viral promotor and polyadenylation signals from the Moloney Murine leukemia virus.

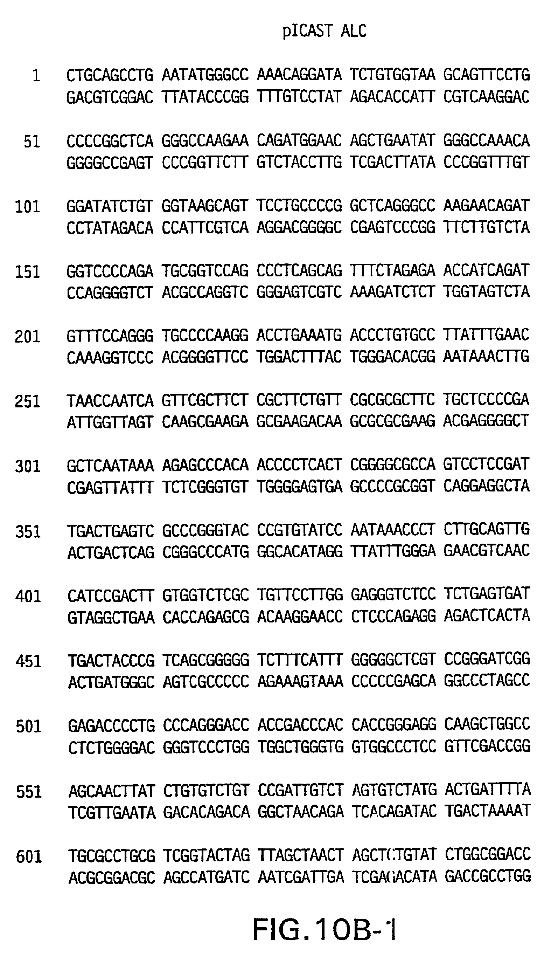

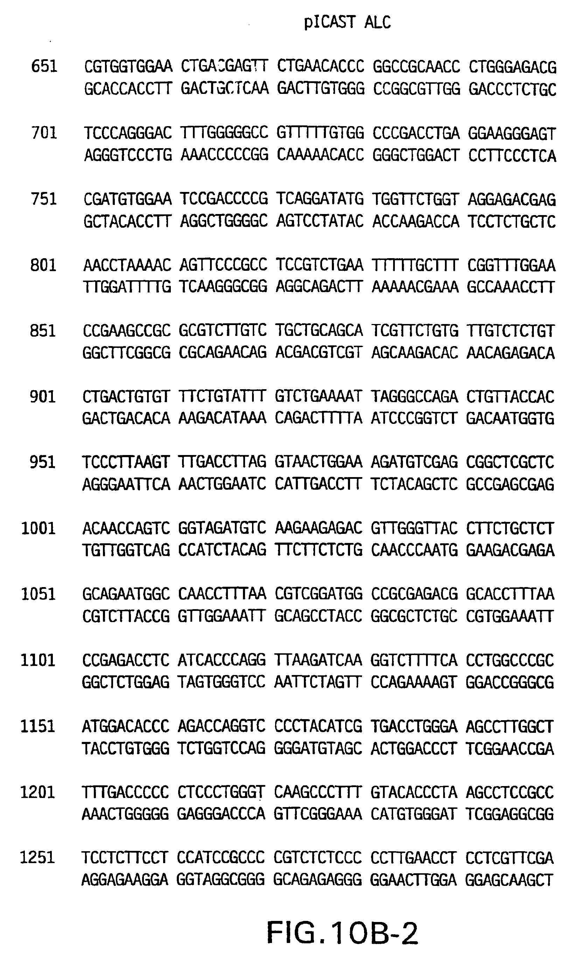

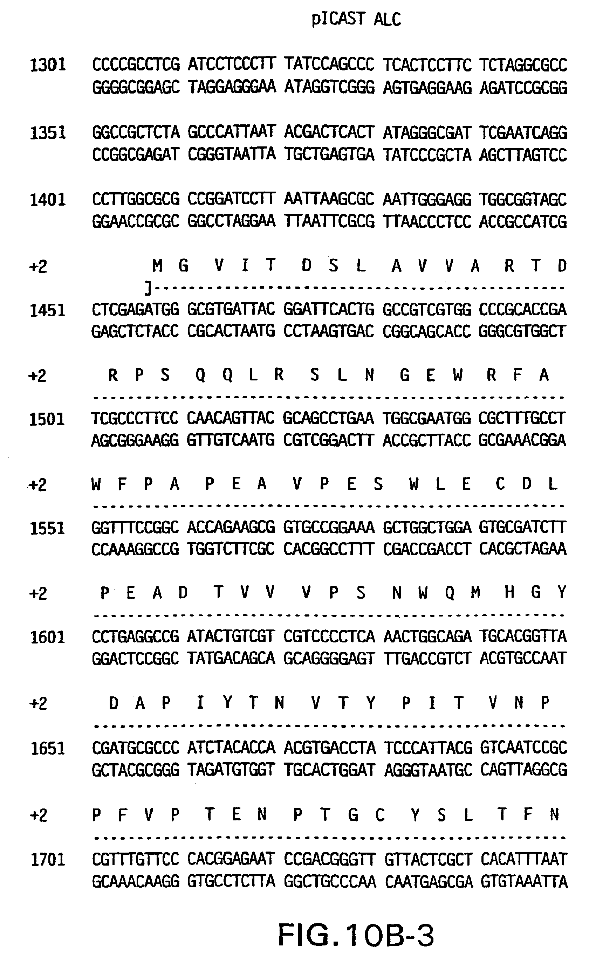

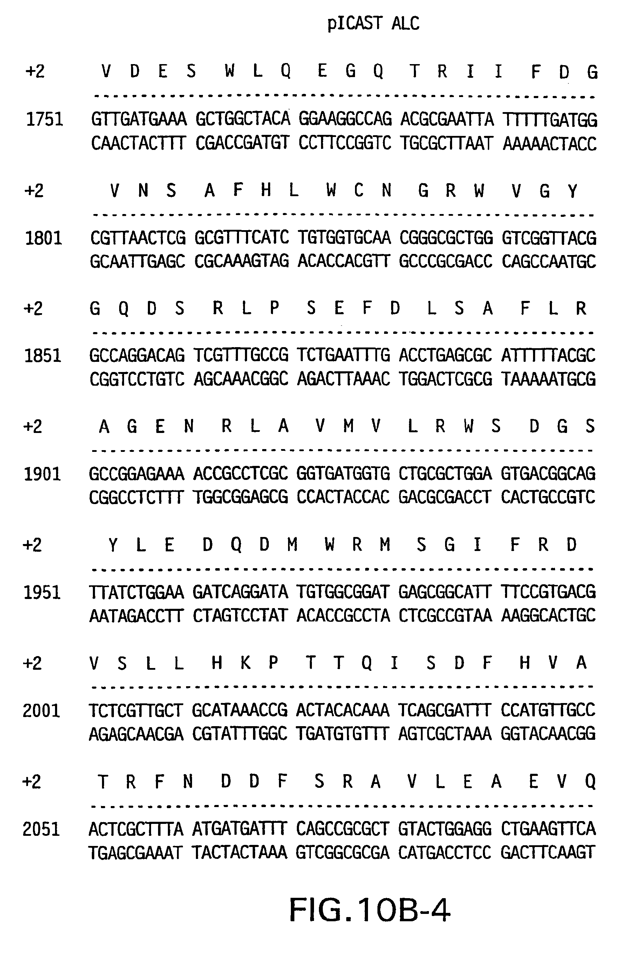

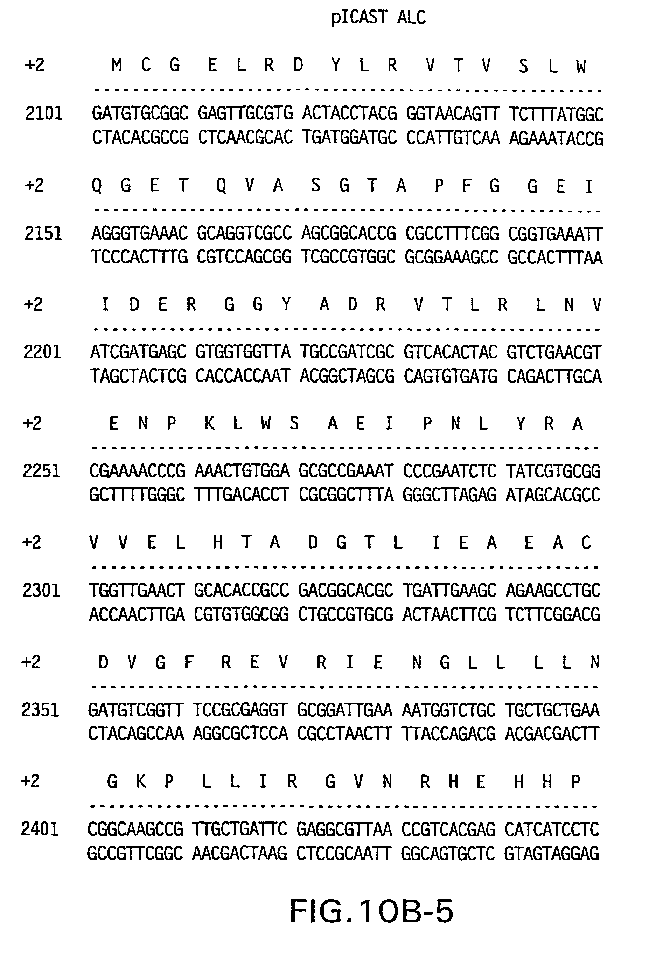

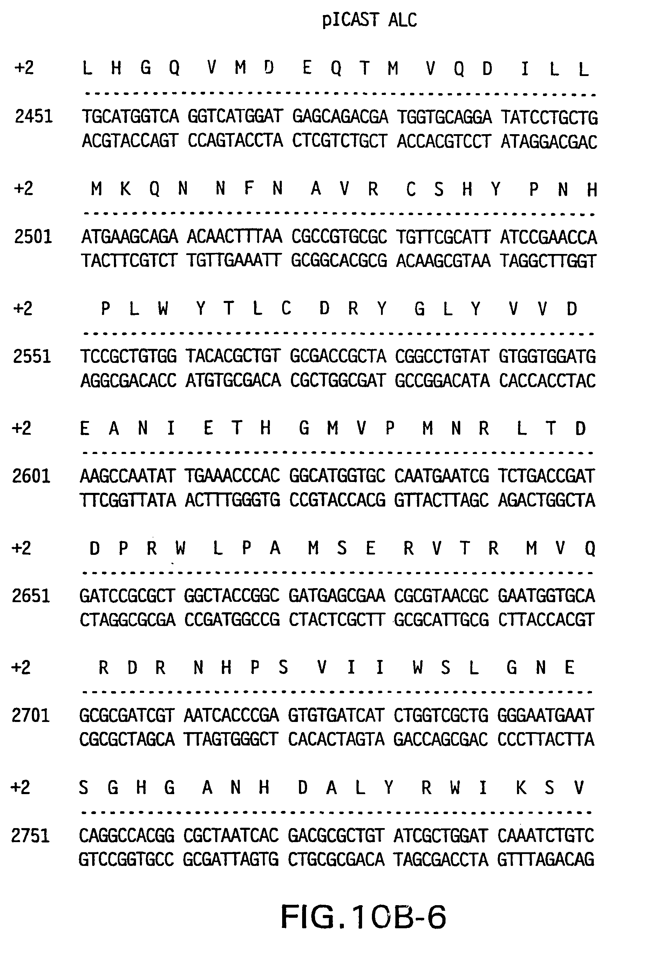

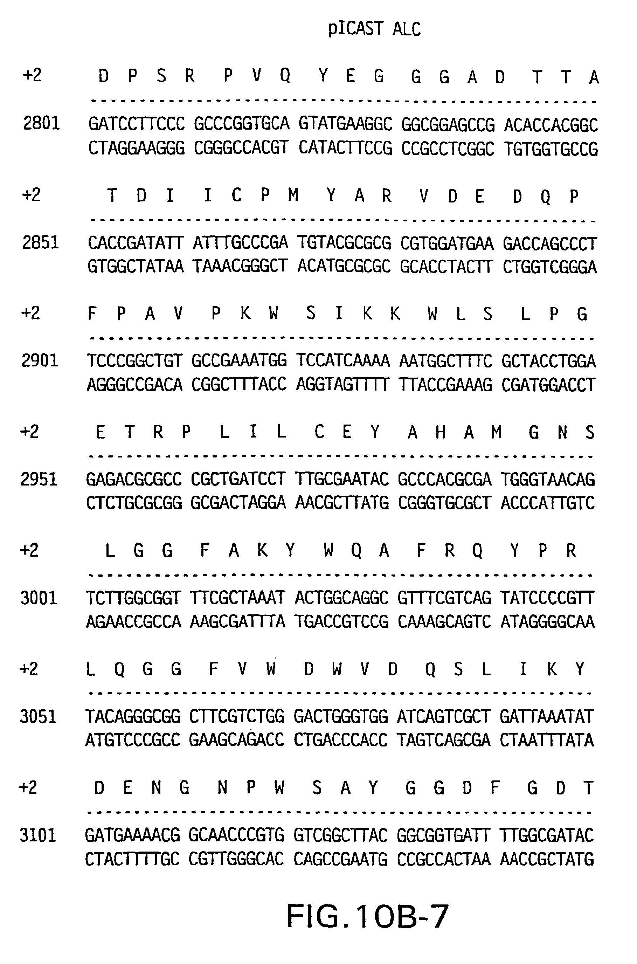

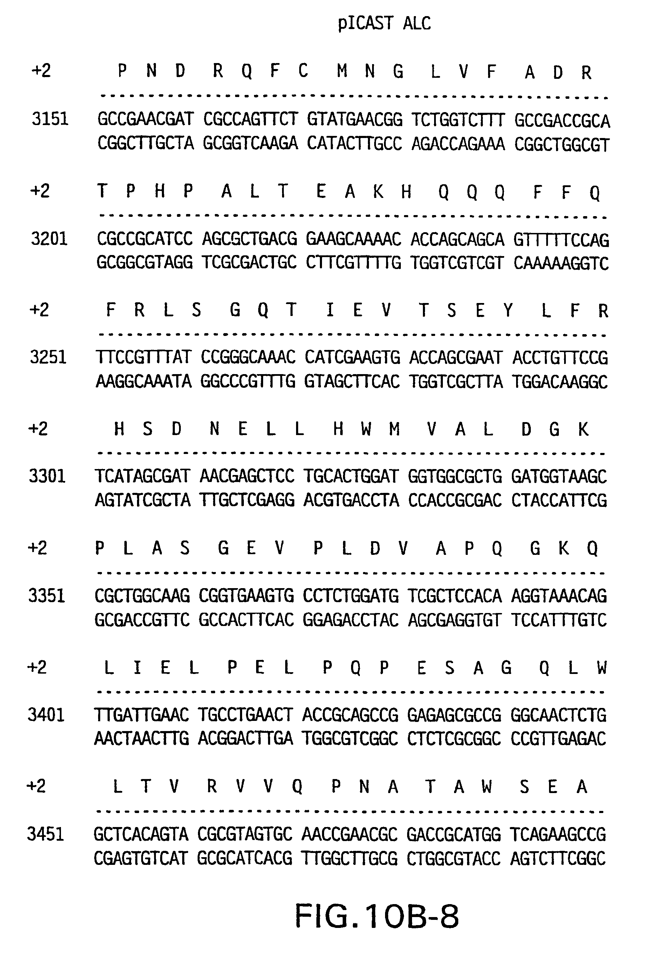

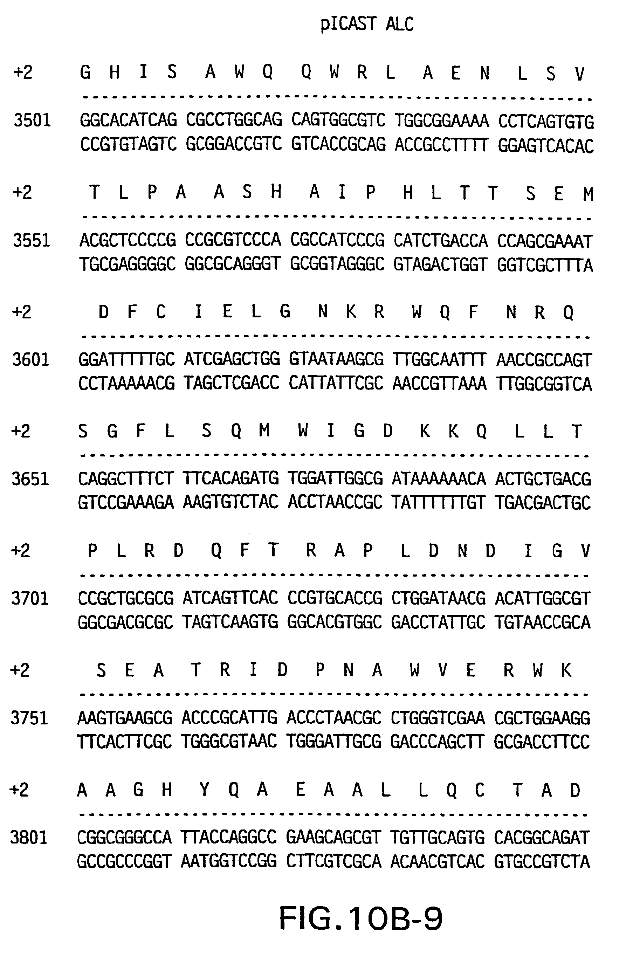

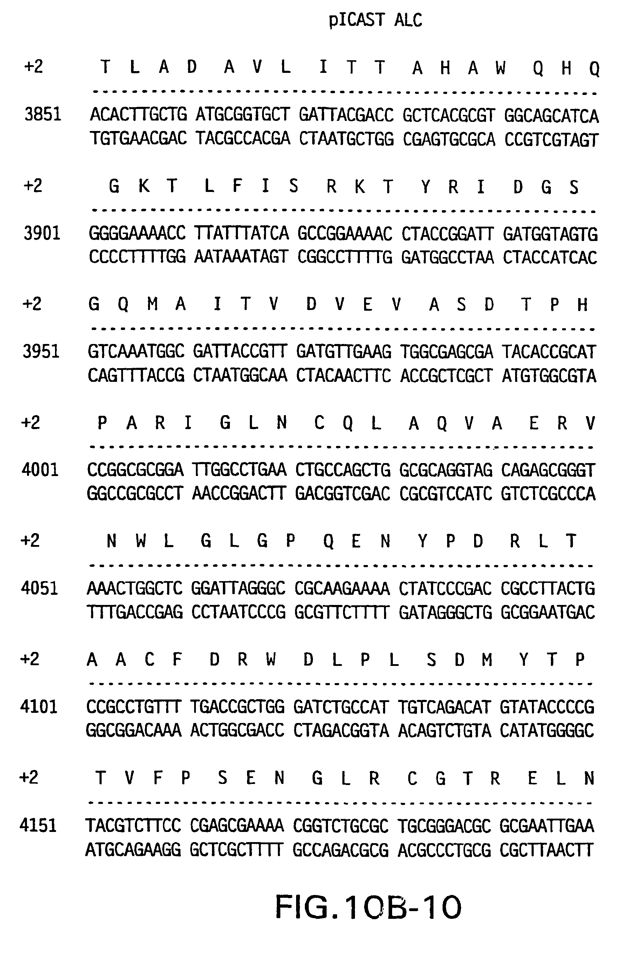

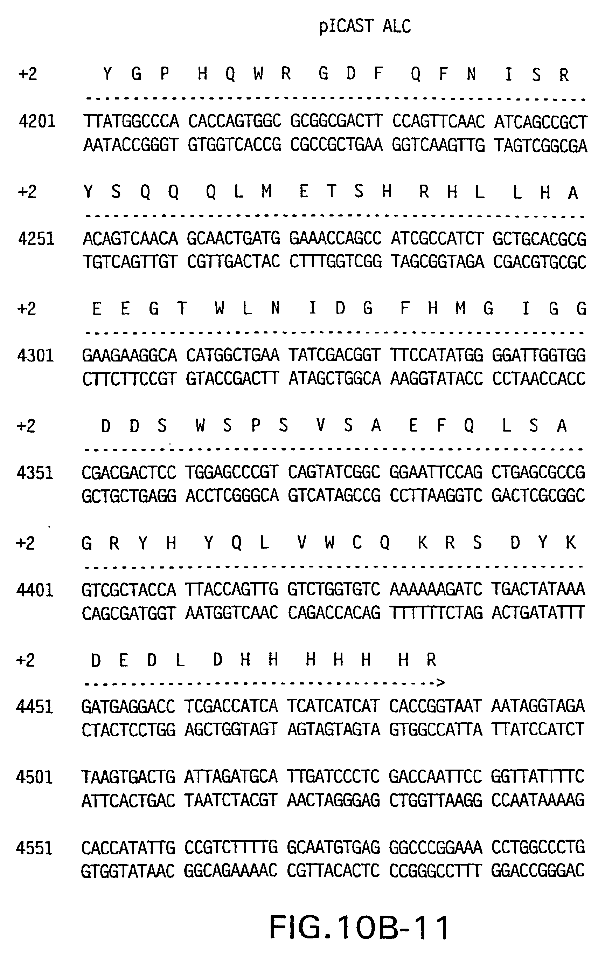

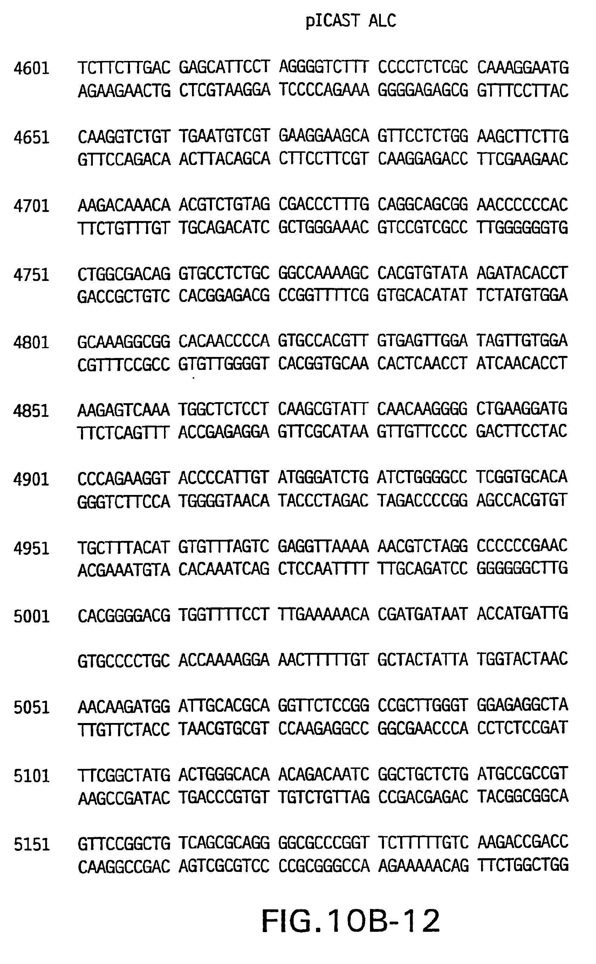

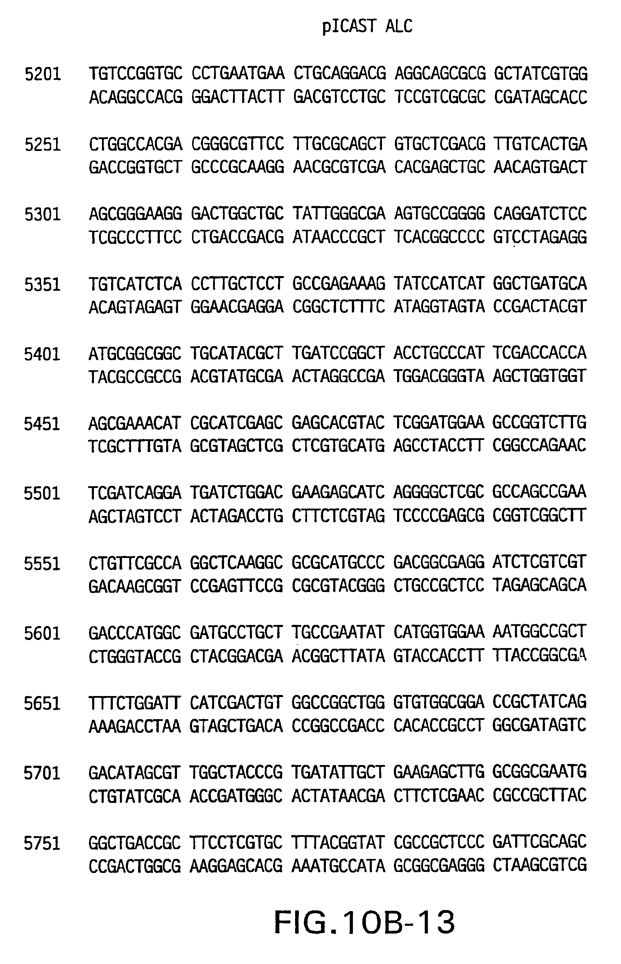

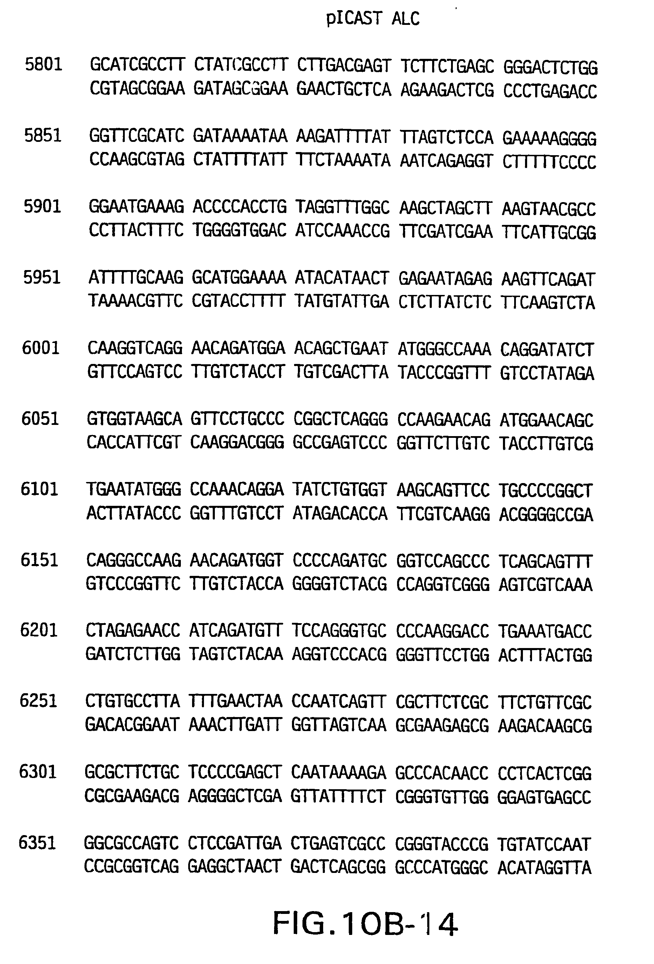

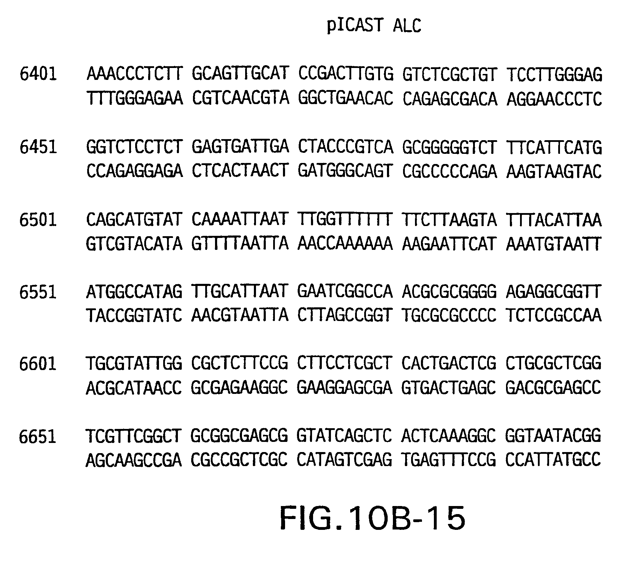

FIGURE 10B. Nucleotide sequence for pICAST ALC.

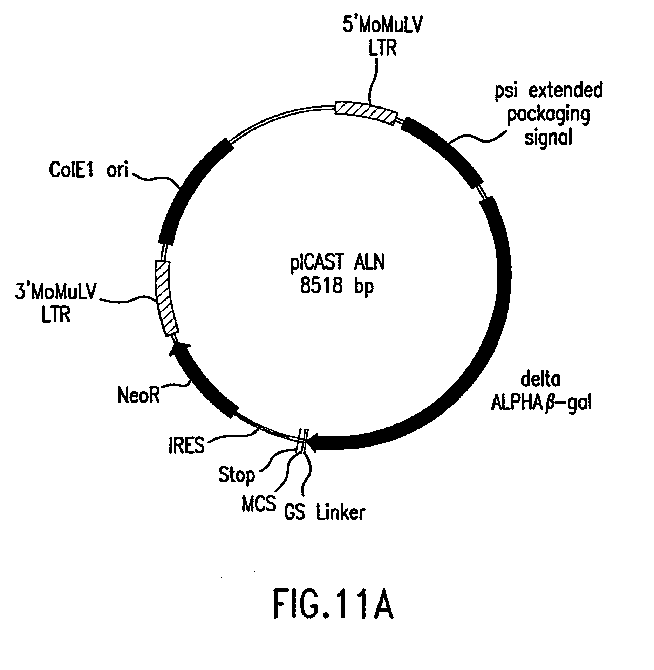

FIGURE 11A. pICAST ALN: Vector for expression of β-galΔα as an N-terminal fusion to the target protein. This construct contains the following features: MCS, multiple cloning site for cloning the target protein in frame with the β-galΔα; GS Linker, (GGGGS)n; NeoR, neomycin resistance gene; IRES, internal ribosome entry site; ColE1ori, origin of replication for growth in E. coli; 5'MoMuLV LTR and 3'MoMuLV LTR, viral promotor and polyadenylation signals from the Moloney Murine leukemia virus.

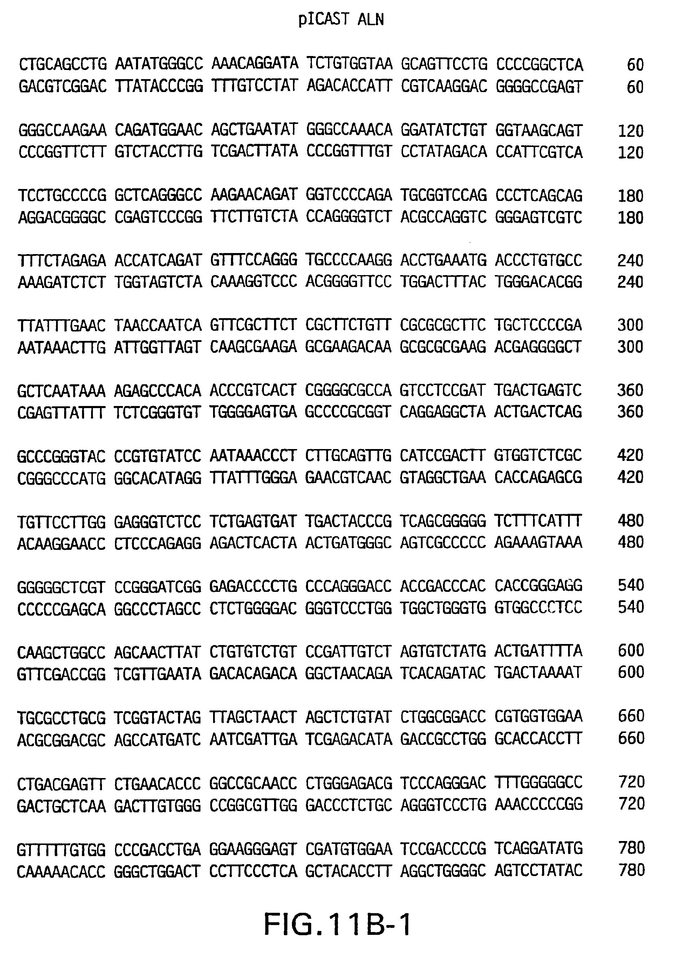

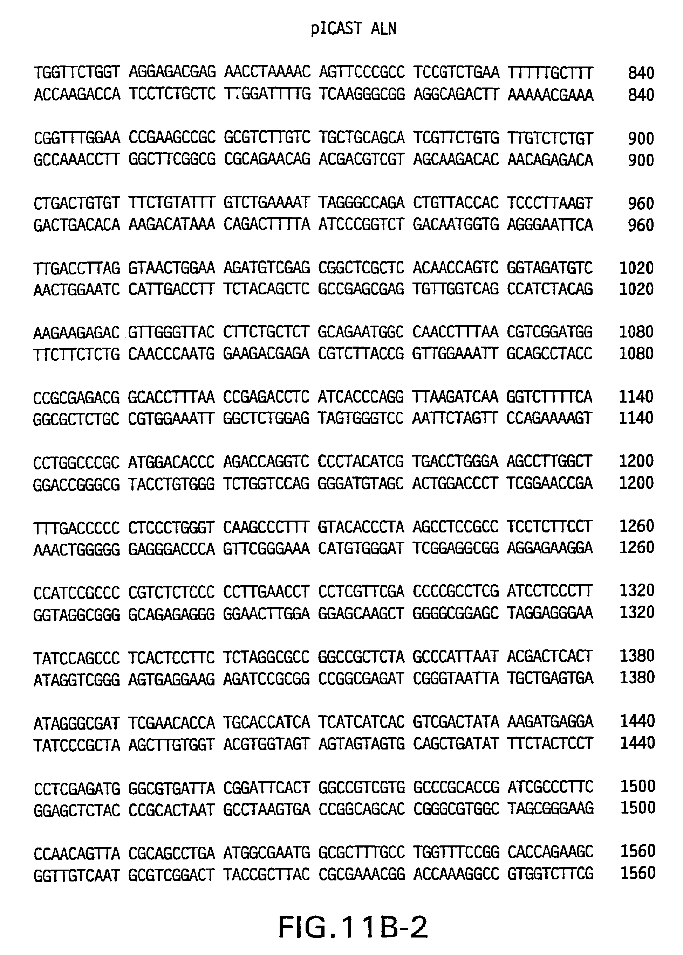

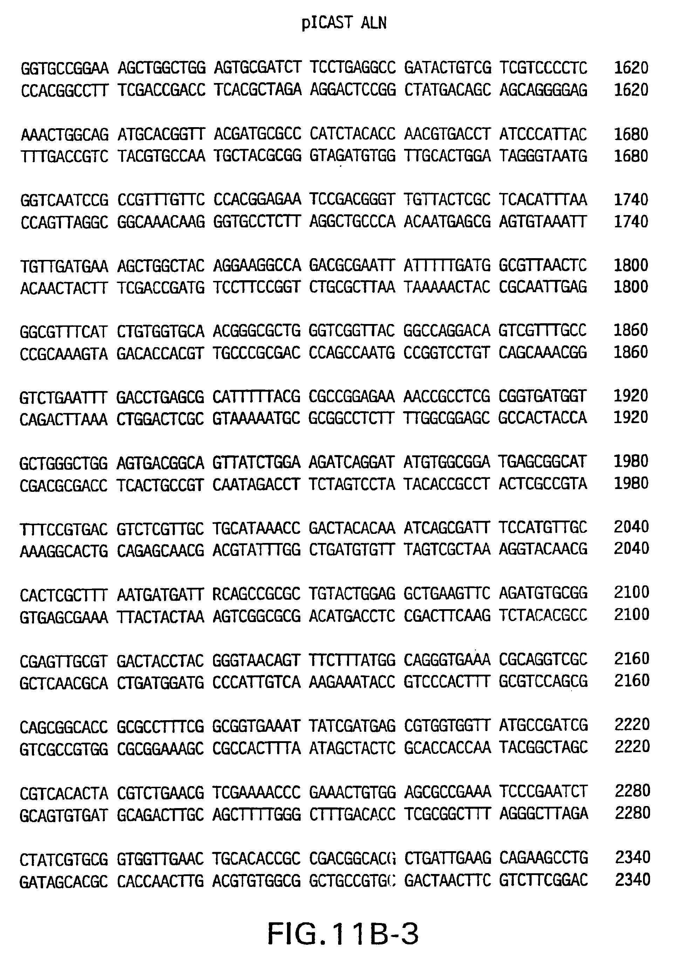

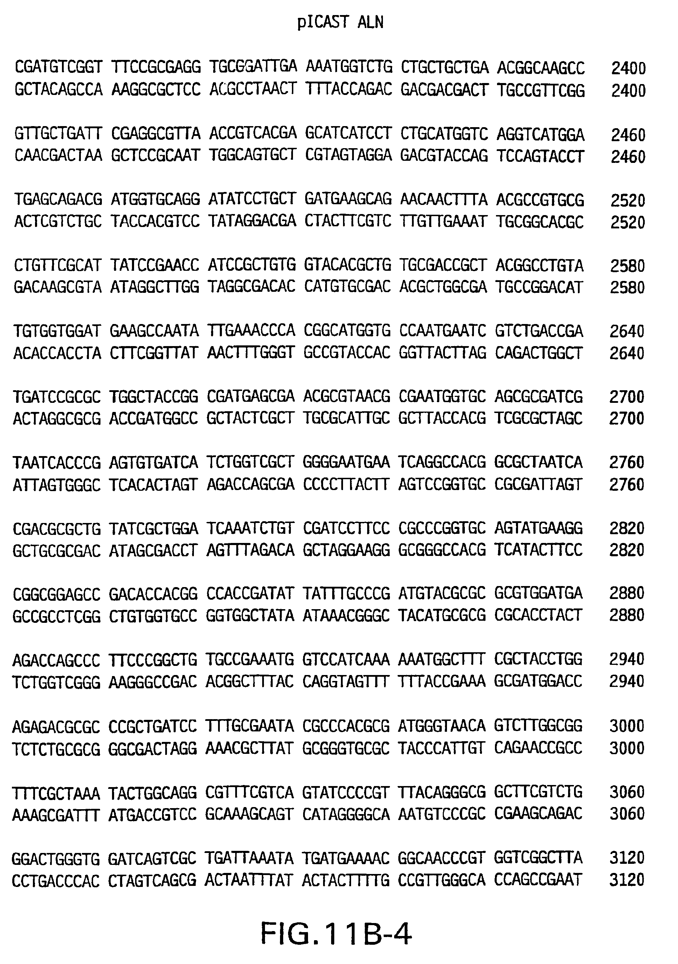

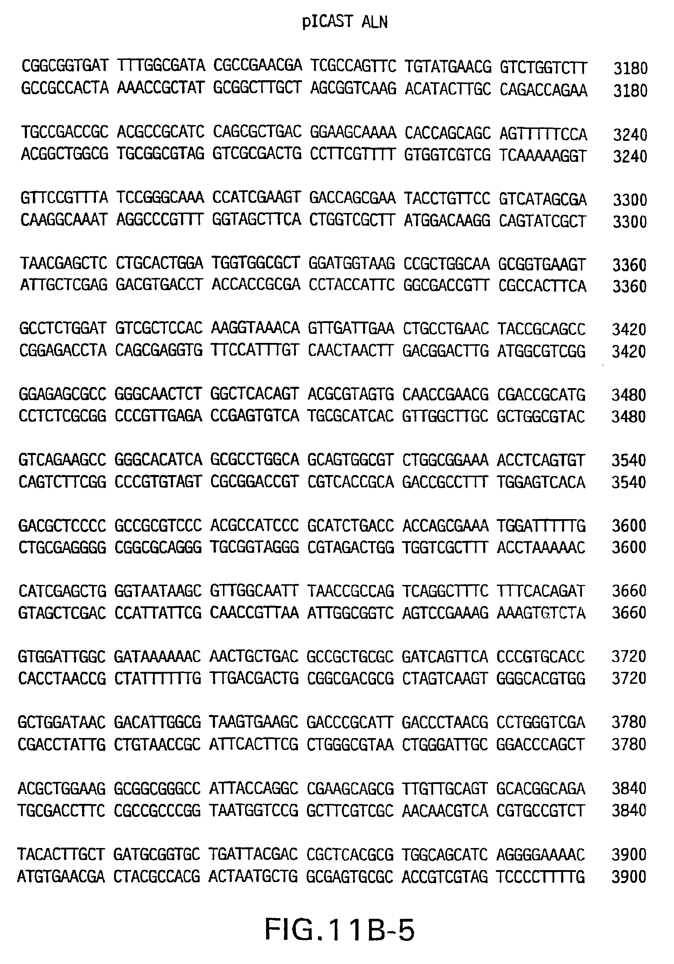

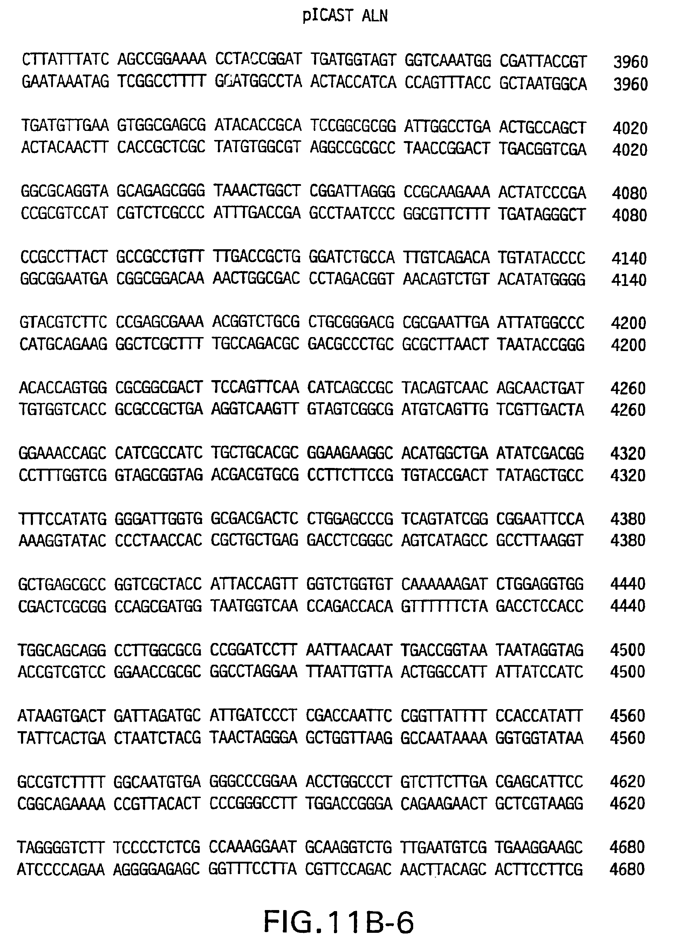

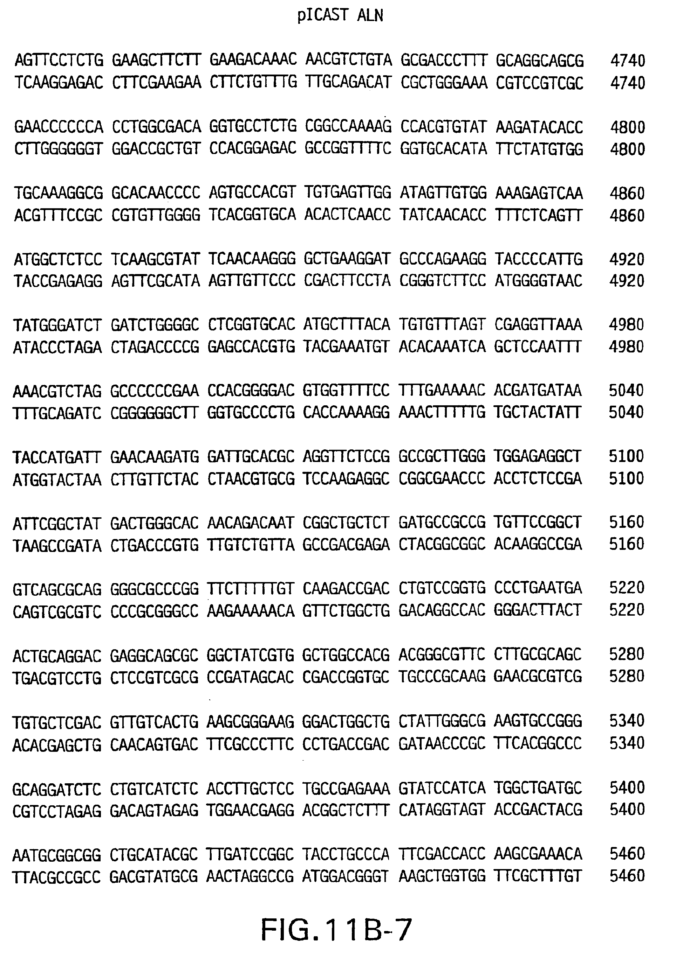

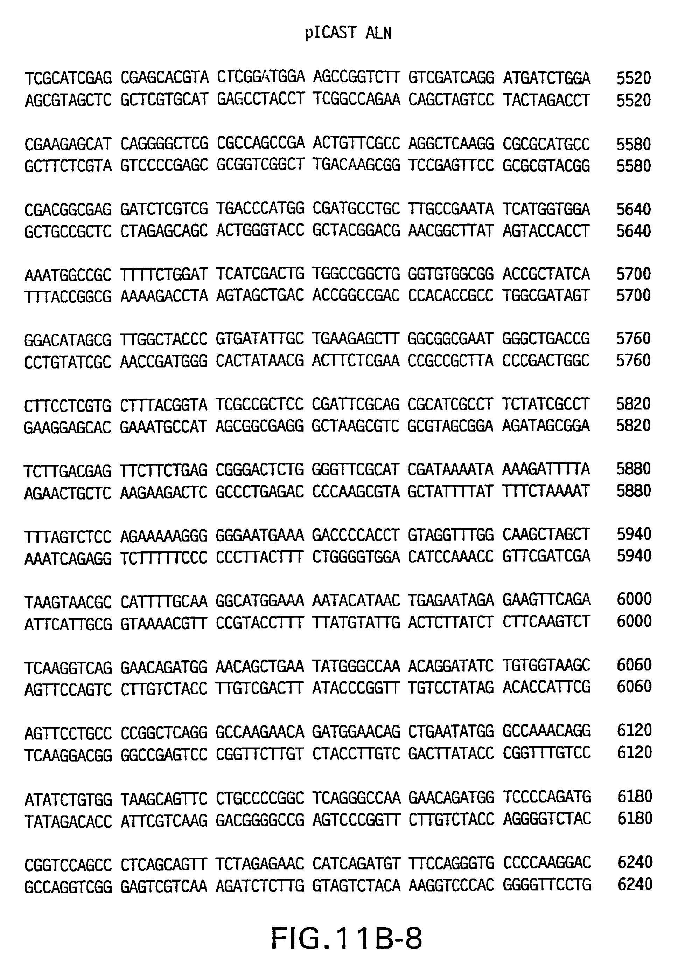

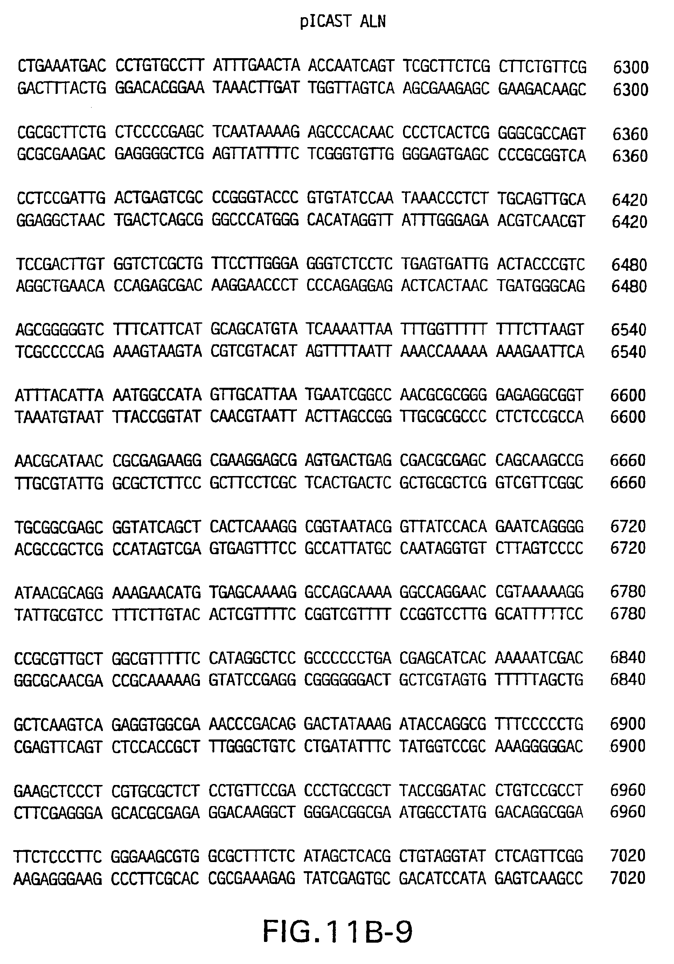

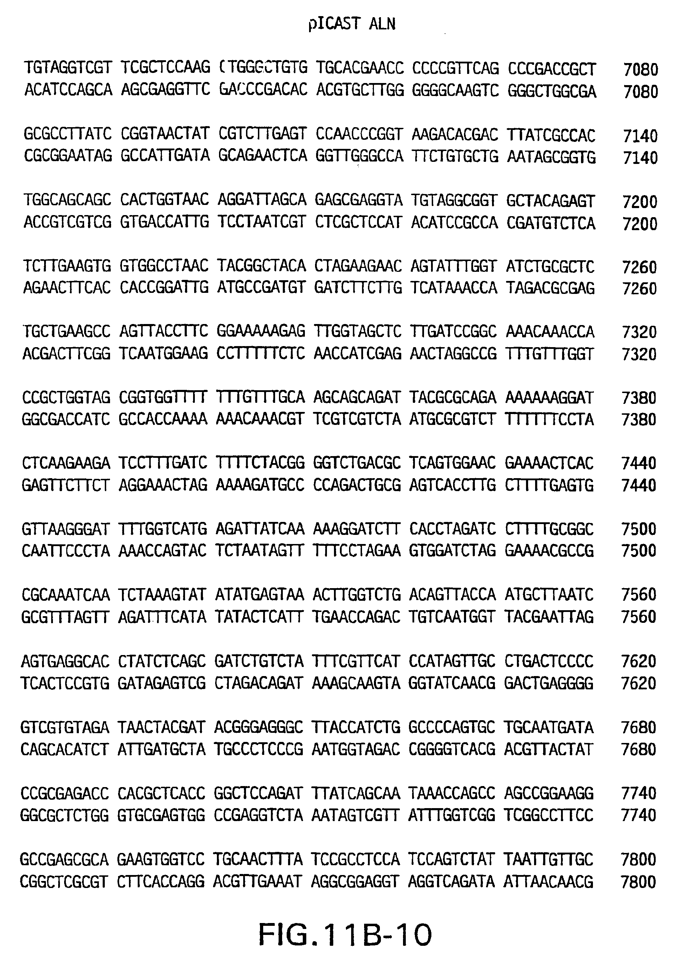

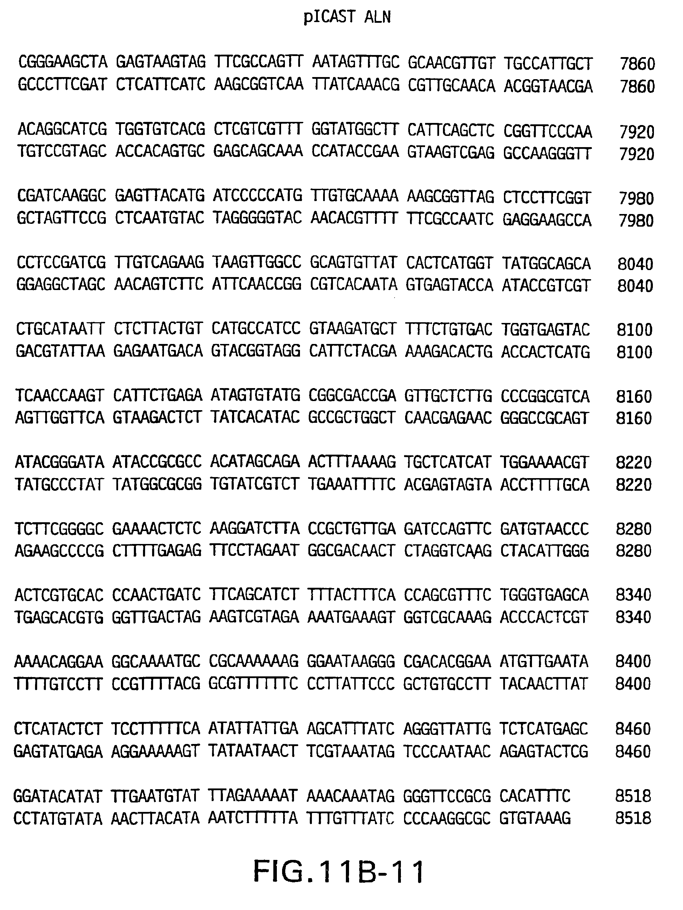

FIGURE 11B. Nucleotide sequence for pICAST ALN.

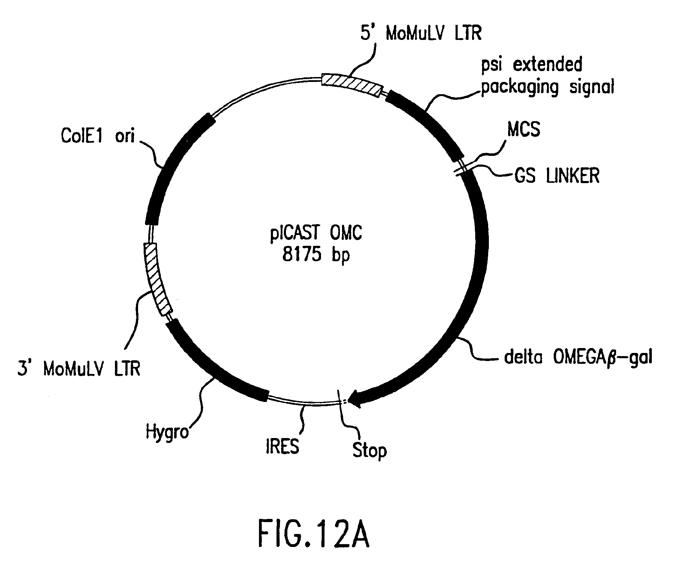

FIGURE 12A. pICAST OMC: Vector for expression of β-galΔω as a C-terminal fusion to the target protein. This construct contains the following features: MCS, multiple cloning site for cloning the target protein in frame with the β-galΔω; GS Linker, (GGGGS)n; Hygro, hygromycin resistance gene; IRES, internal ribosome entry site; ColE1ori, origin of replication for growth in E. coli; 5'MoMuLV LTR and 3'MoMuLV LTR, viral promotor and polyadenylation signals from the Moloney Murine leukemia virus.

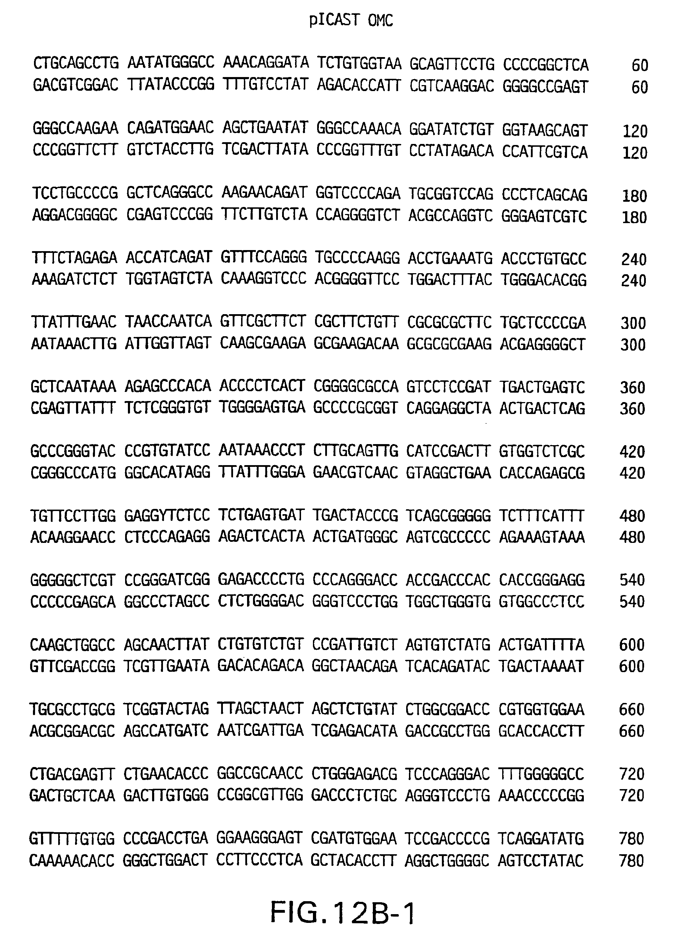

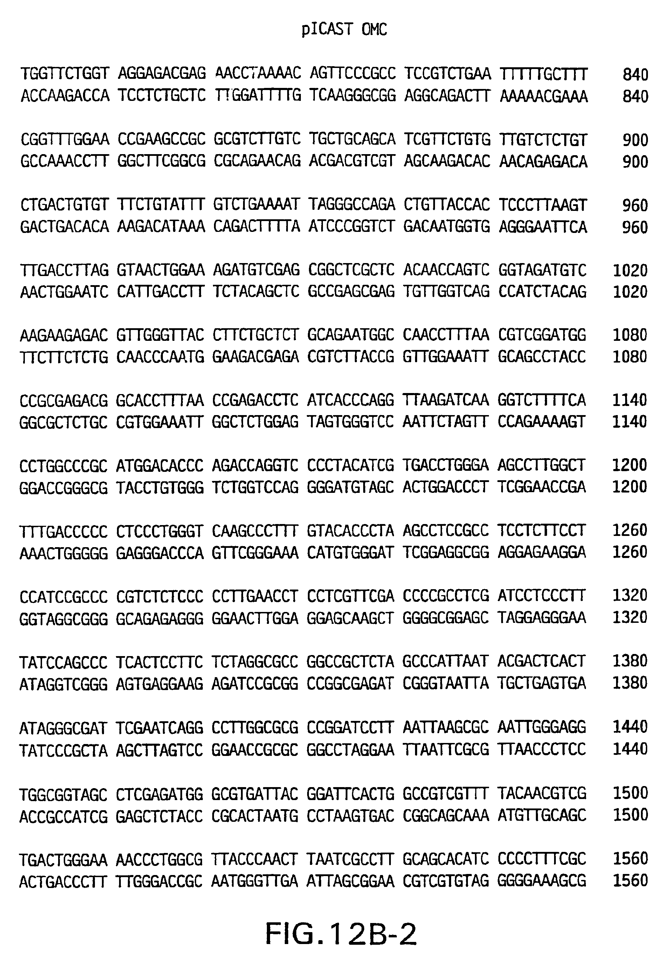

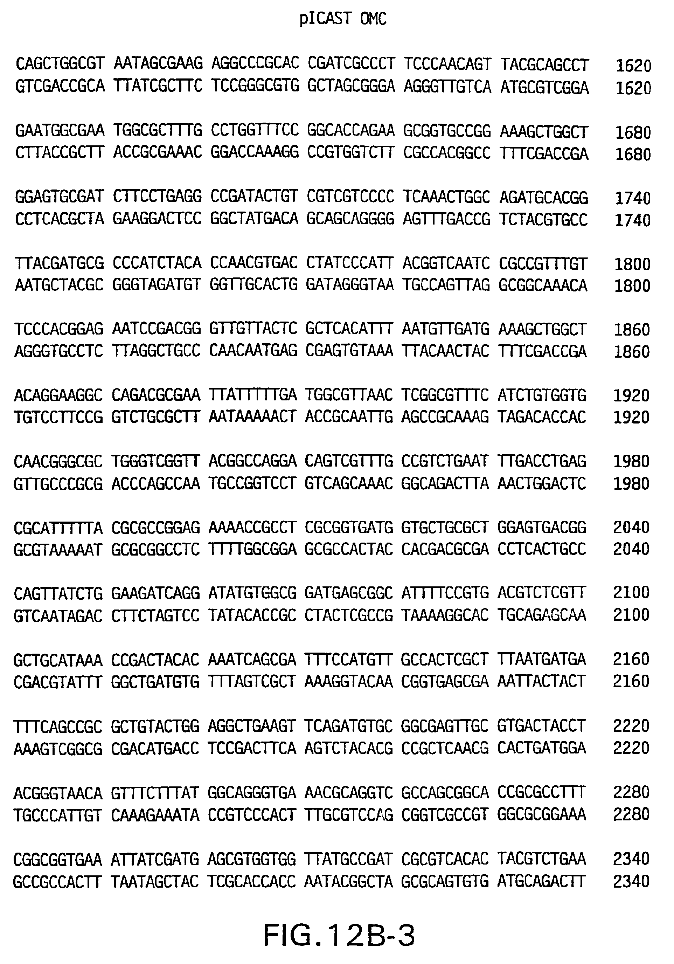

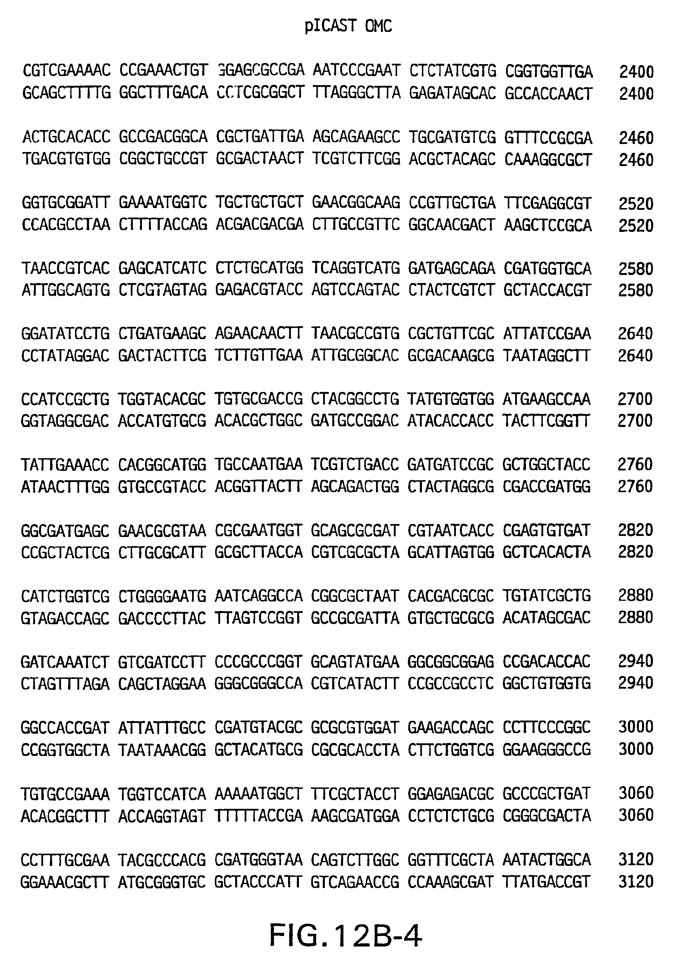

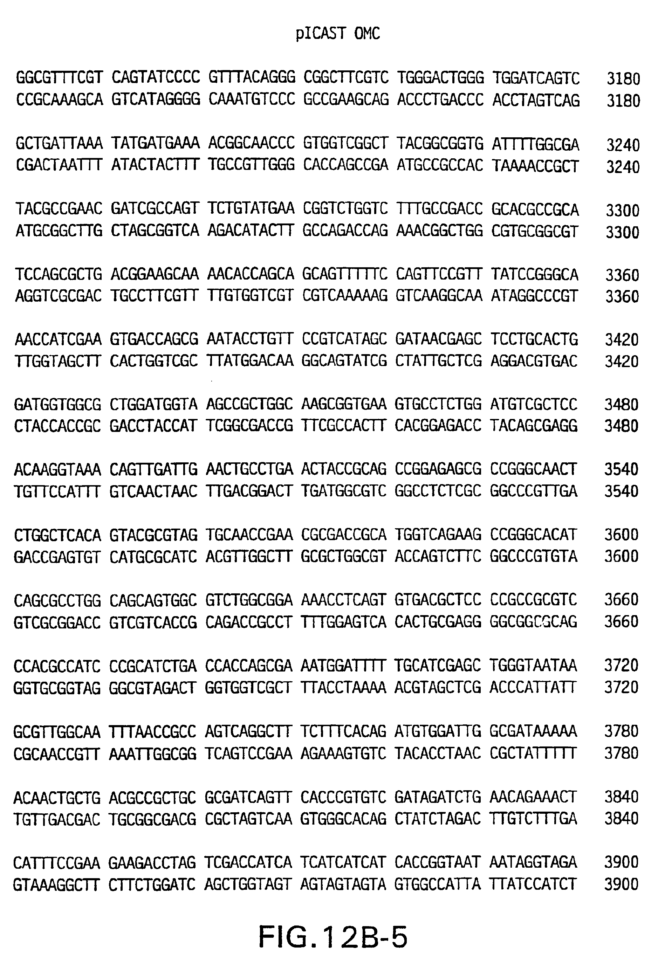

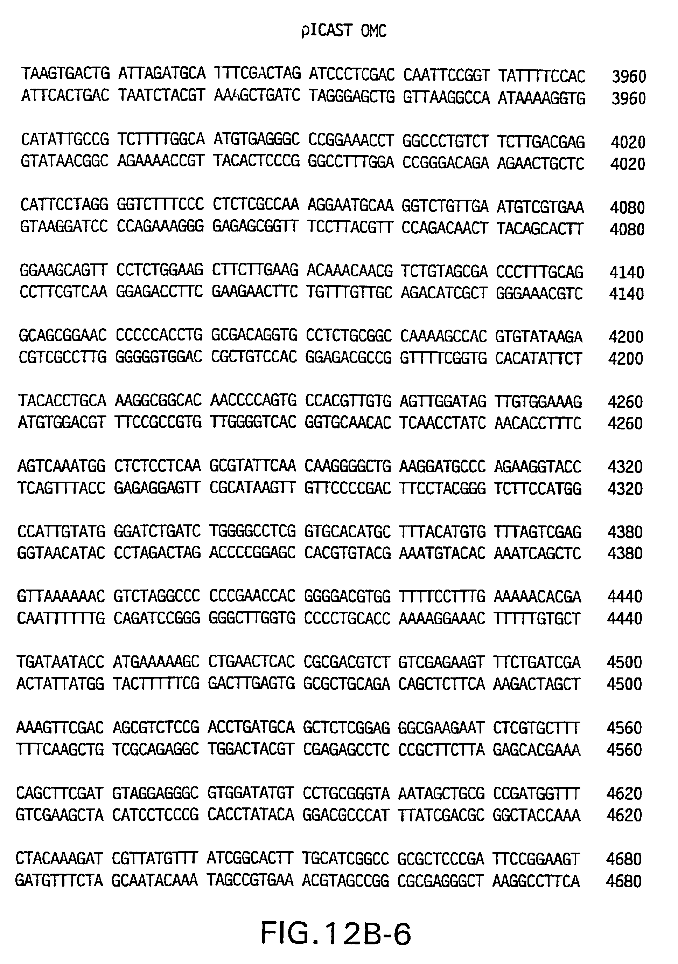

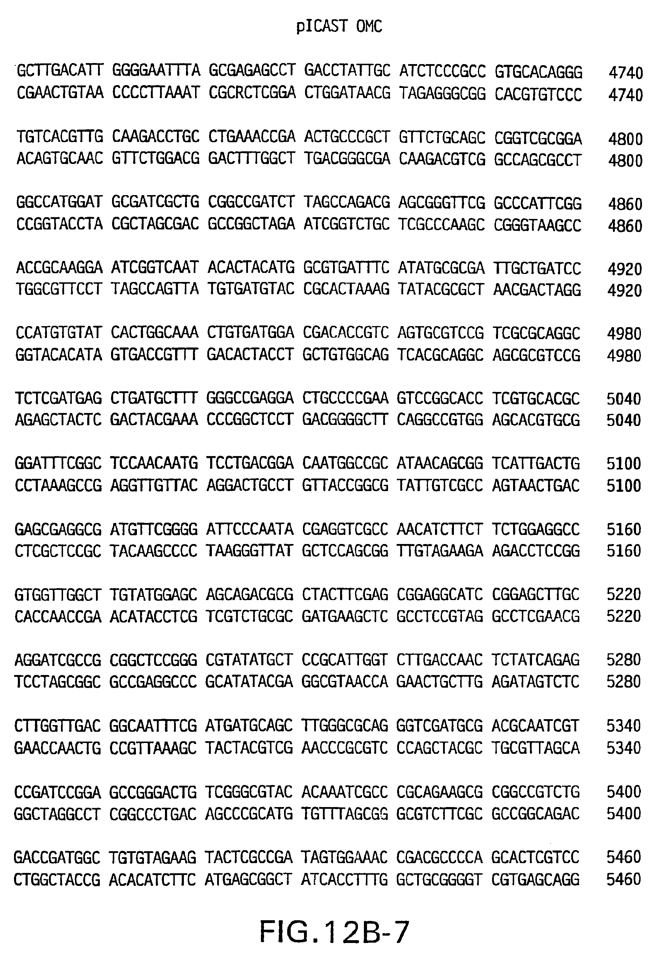

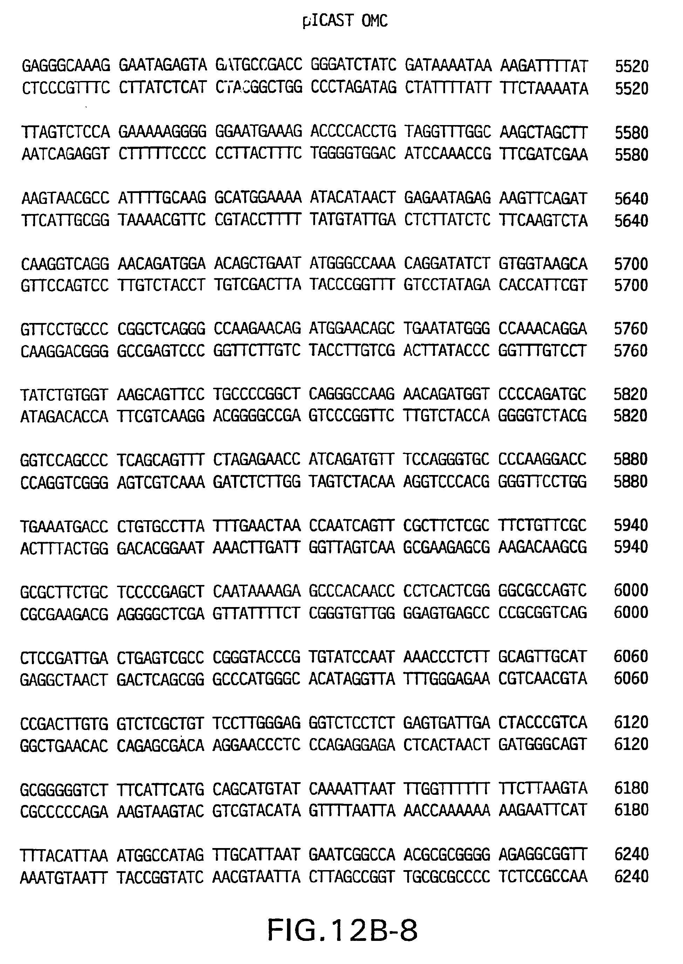

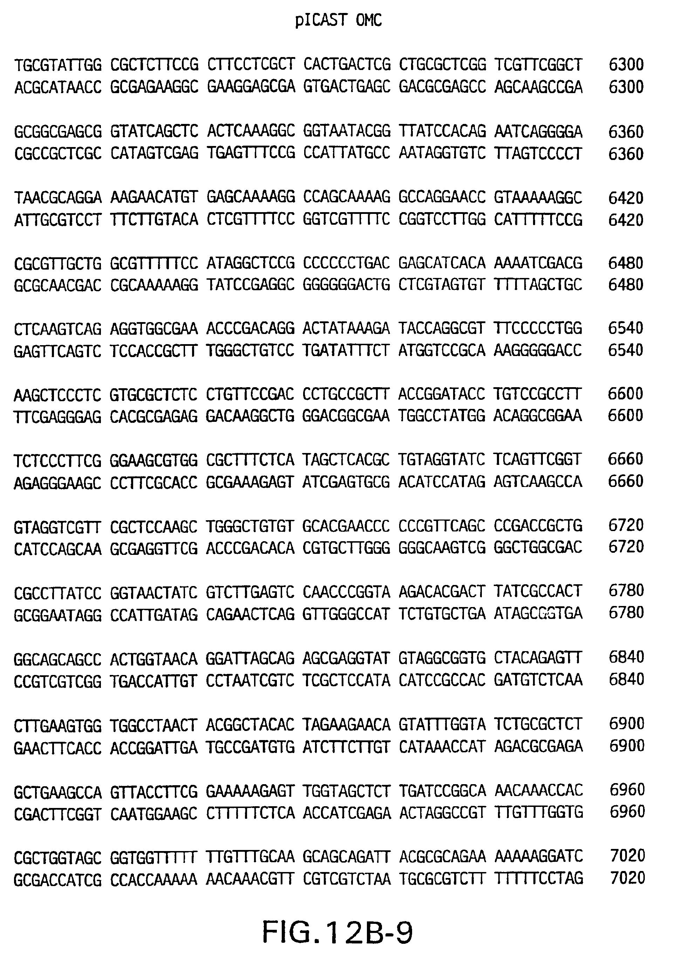

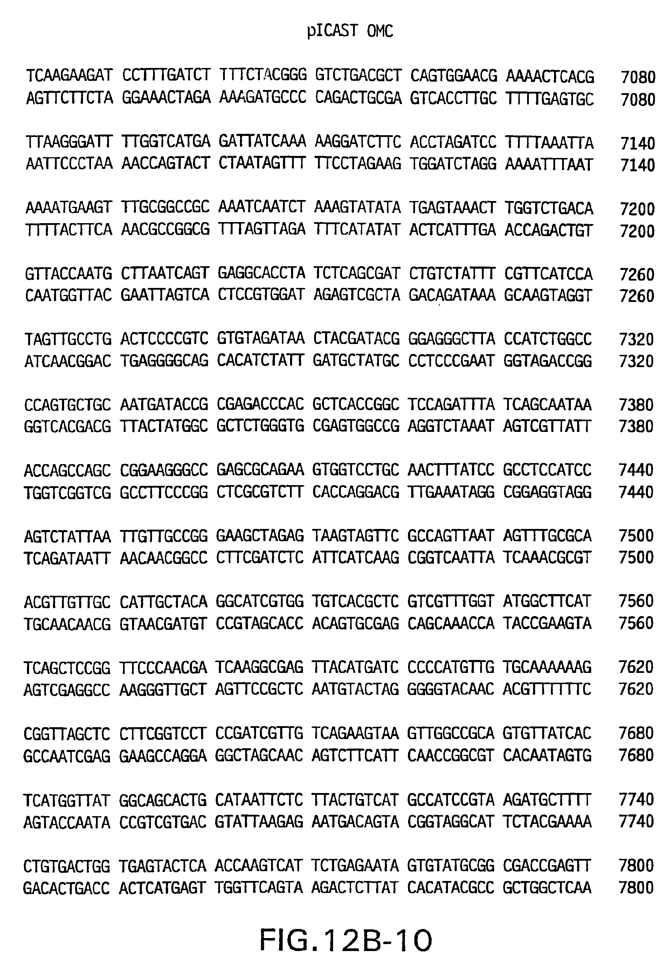

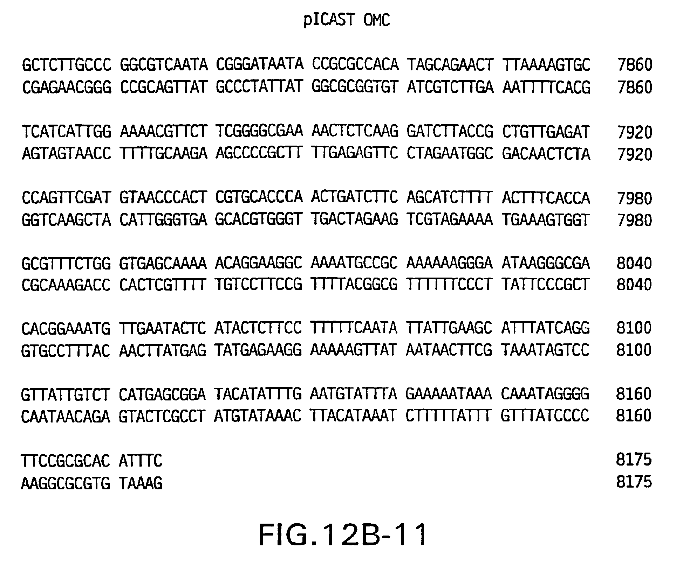

FIGURE 12B. Nucleotide sequence for pICAST OMC.

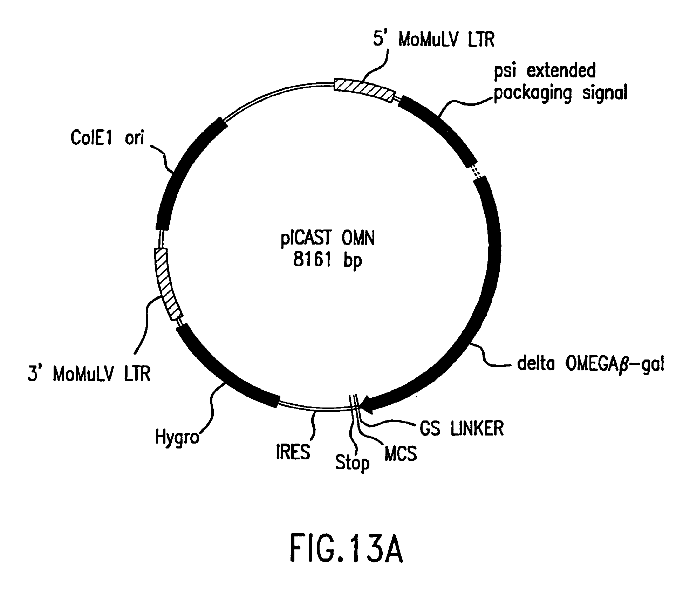

FIGURE 13A. pICAST OMN: Vector for expression of β-galΔω as an N-terminal fusion to the target protein. This construct contains the following features: MCS, multiple cloning site for cloning the target protein in frame with the β-galΔω; GS Linker, (GGGGS)n; Hygro, hygromycin resistance gene; IRES, internal ribosome entry site; ColE1ori, origin of replication for growth in E. coli; 5'MoMuLV LTR and 3'MoMuLV LTR, viral promotor and polyadenylation signals from the Moloney Murine leukemia virus.

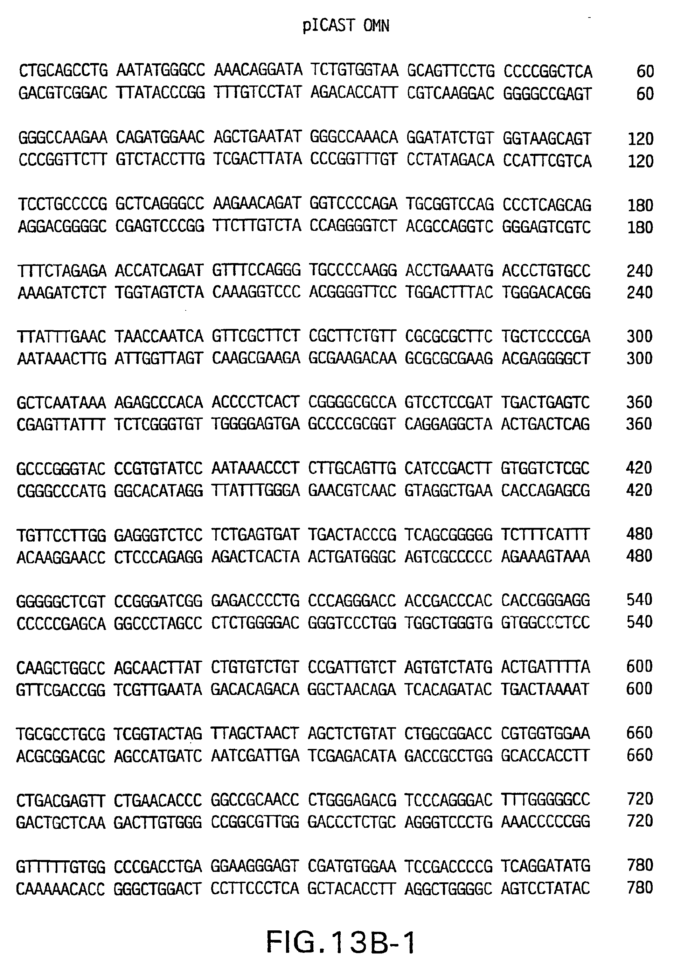

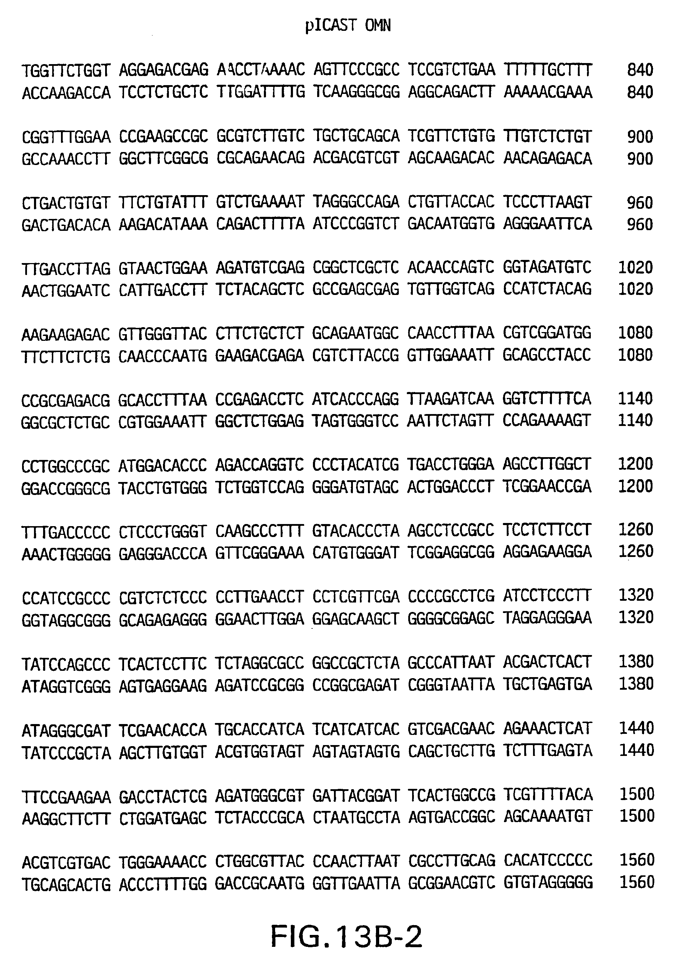

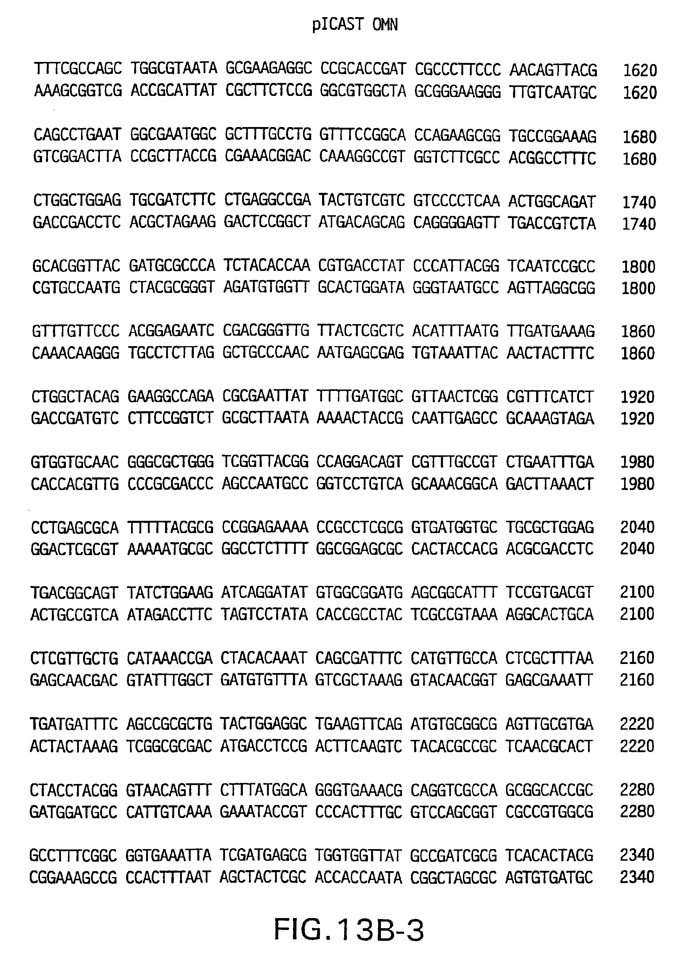

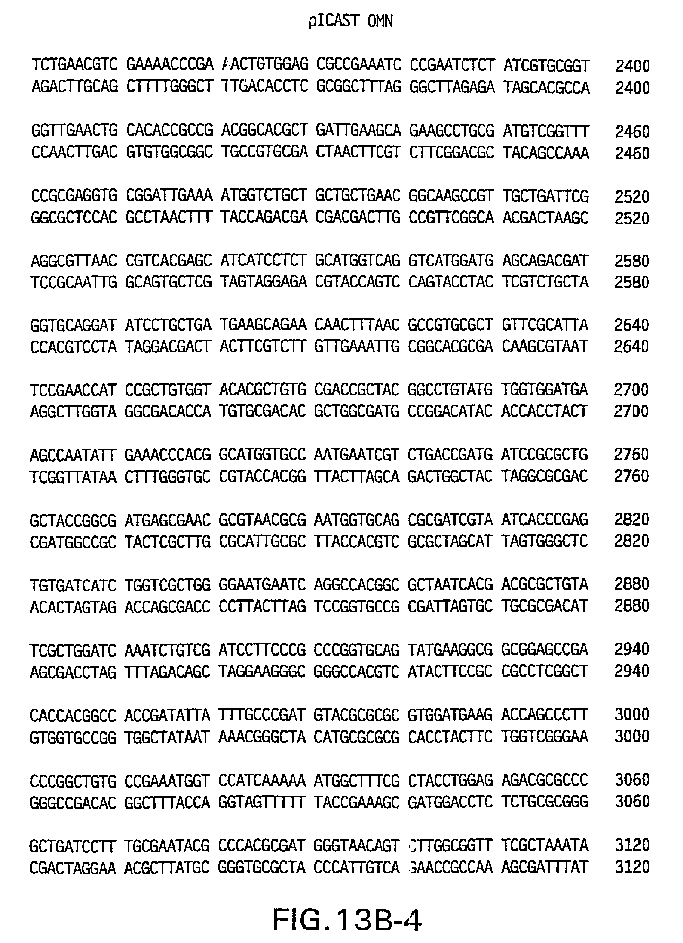

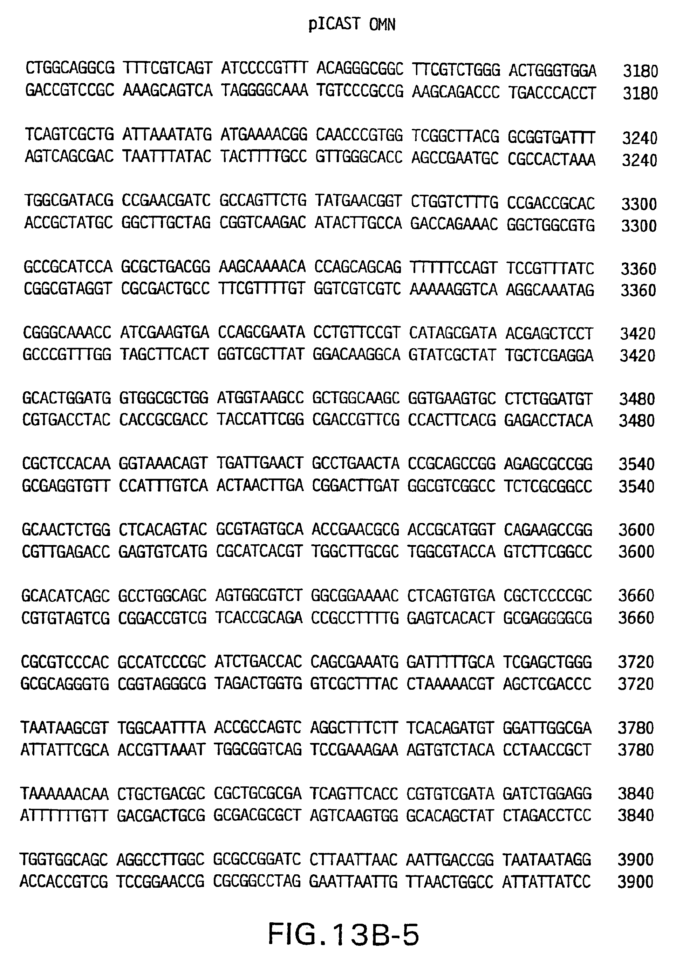

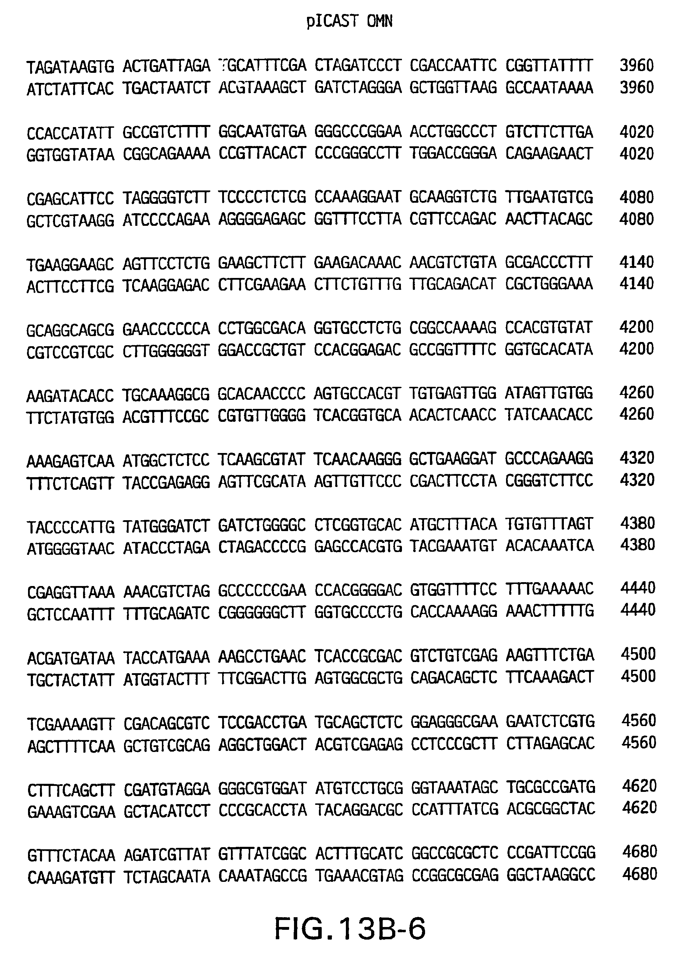

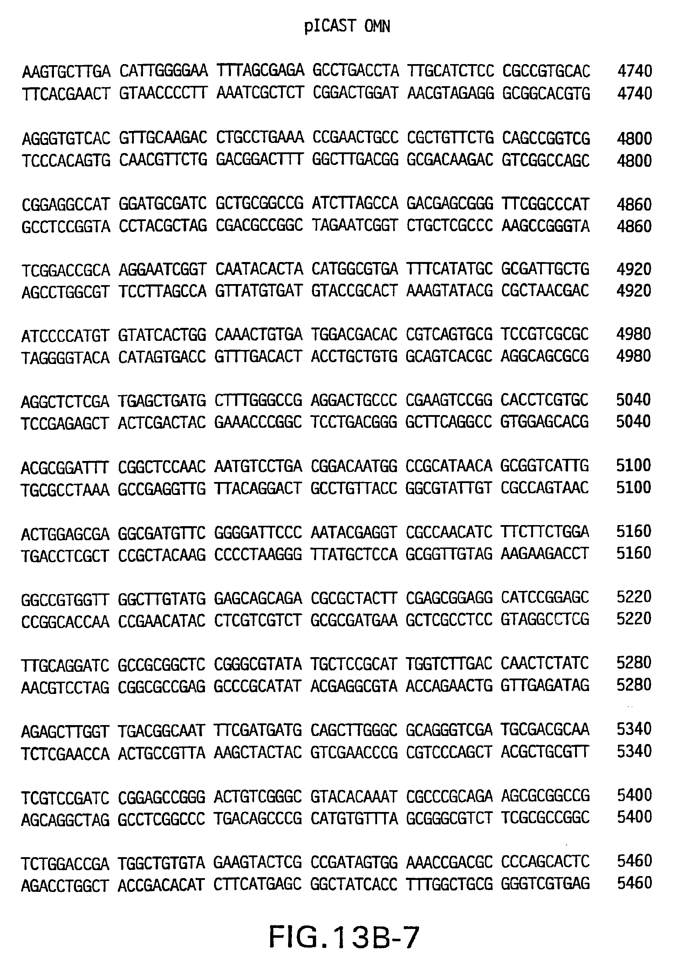

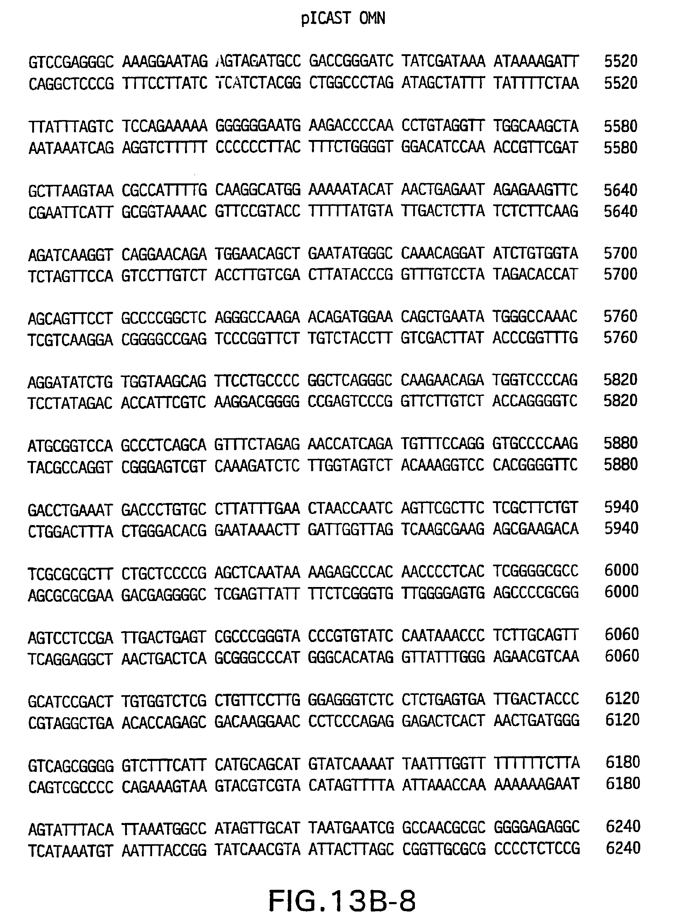

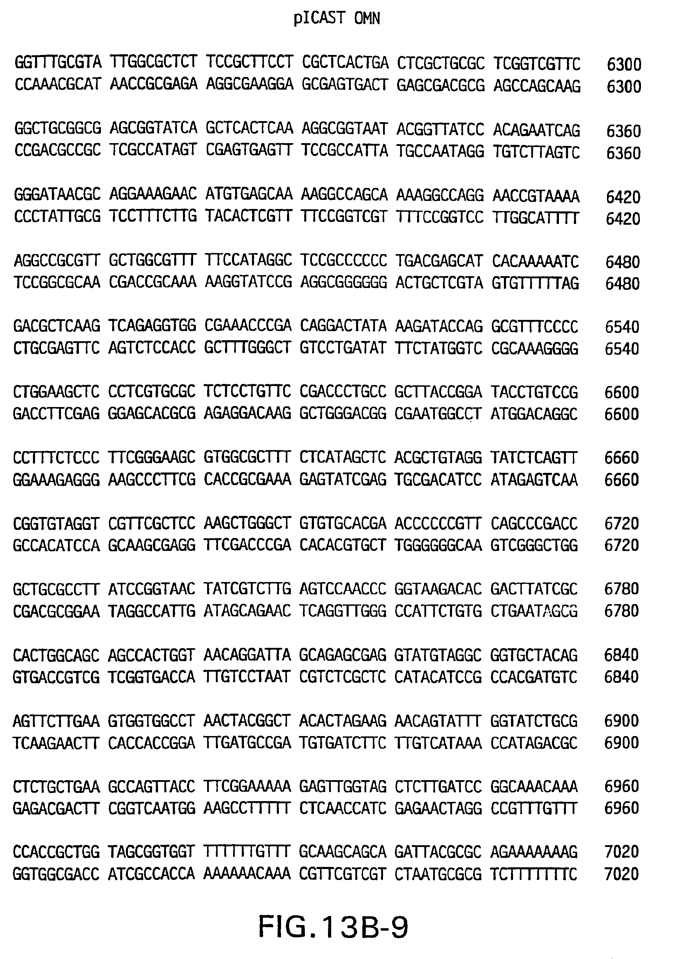

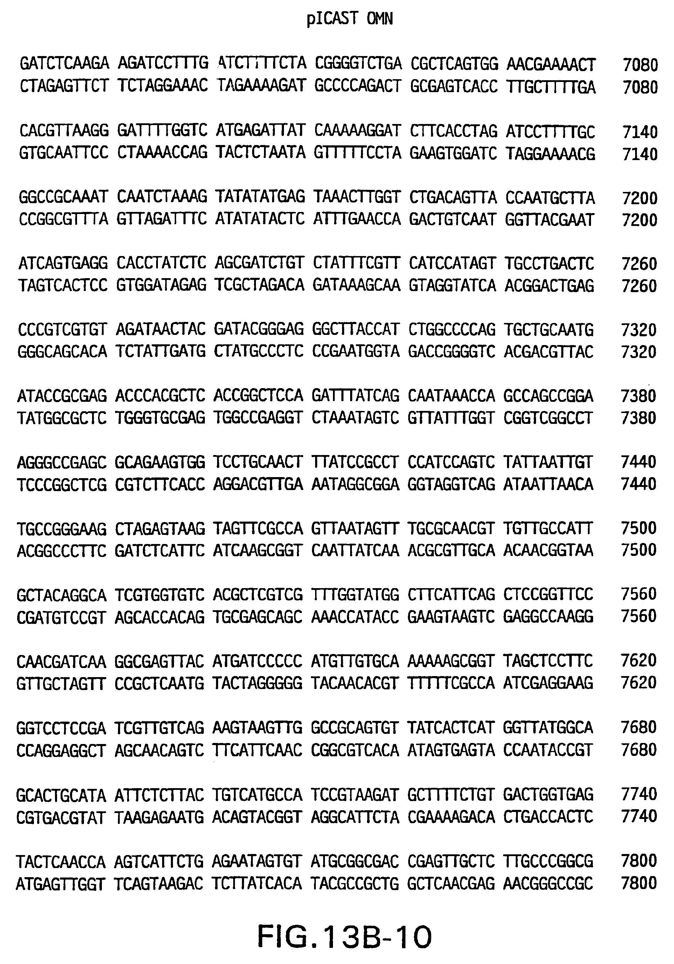

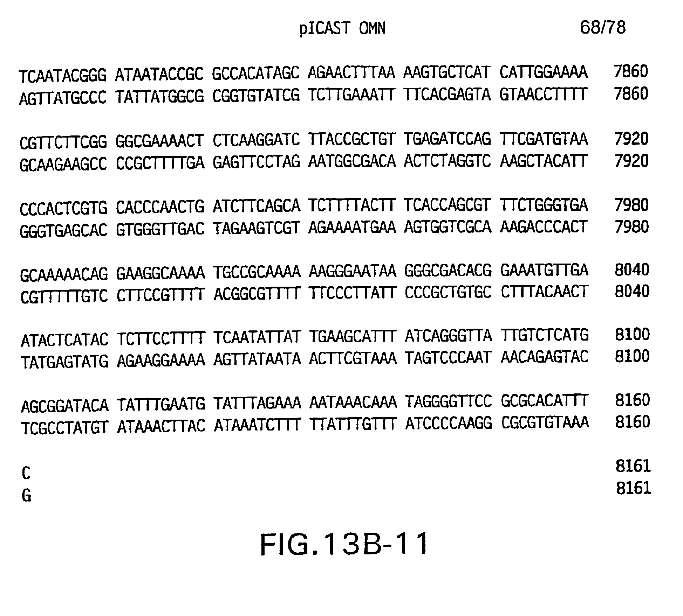

FIGURE 13B. Nucleotide sequence for pICAST OMN.

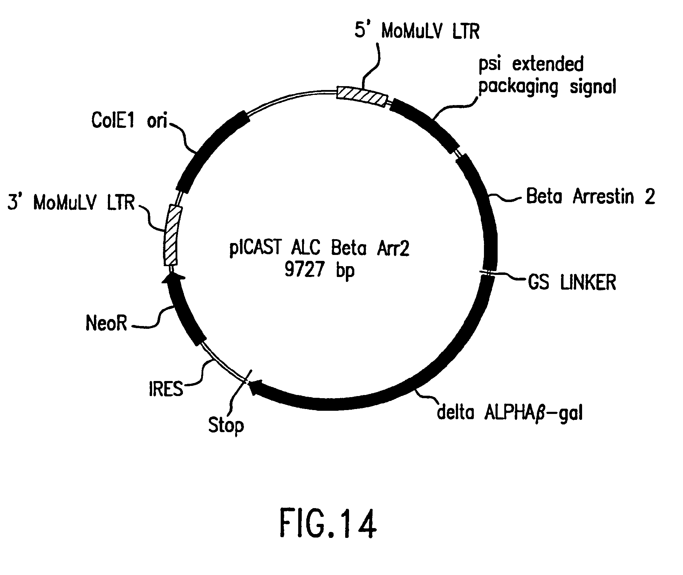

FIGURE 14. pICAST ALC βArr2: Vector for expression of β-galΔα as a C-terminal fusion to β-arrestin-2. The coding sequence of human β-arrestin-2 (Genebank Accession Number: NM_004313) was cloned in frame to β-galΔα in a pICAST ALC vector.
FIGURE 15. pICAST OMC βArr2: Vector for expression of β-galΔω as a C-terminal fusion to β-arrestin-2. The coding sequence of human β-arrestin-2 (Genebank Accession Number: NM_004313) was cloned in frame to β-galΔω in a pICAST OMC vector.

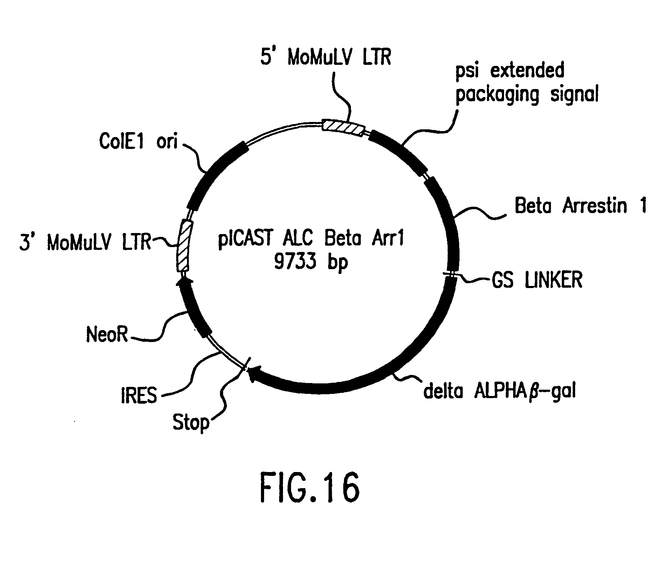

FIGURE 16. pICAST ALC βArr1 : Vector for expression of β-galΔα as a C-terminal fusion to β-arrestin-1. The coding sequence of human β-arrestin-1 (Genebank Accession Number: NM_004041) was cloned in frame to β-galΔα in a pICAST ALC vector.

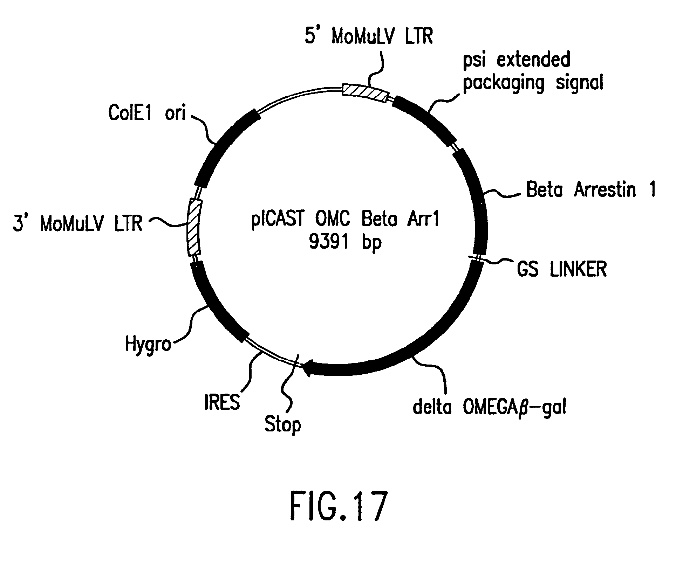

FIGURE 17. pICAST OMC βArr1: Vector for expression of β-galΔω as a C-terminal fusion to β-arrestin-1. The coding sequence of human β-arrestin-1 (Genebank Accession Number: NM_004041) was cloned in frame to β-galΔω in a pICAST OMC vector.
FIGURE 18. pICAST ALC β2AR: Vector for expression of β-galΔα as a C-terminal fusion to β2 Adrenergic Receptor. The coding sequence of human β2 Adrenergic Receptor (Genebank Accession Number: NM_000024) was cloned in frame to β-galΔα in a pICAST ALC vector.

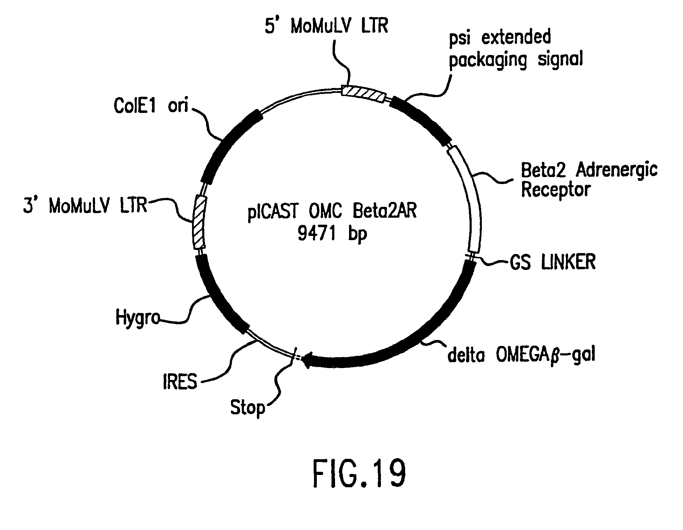

FIGURE 19. pICAST OMC β2AR: Vector for expression of β-galΔω as a C-terminal fusion β2 Adrenergic Receptor. The coding sequence of human β2 Adrenergic Receptor (Genebank Accession Number: NM_000024) was cloned in frame to β-galΔω in a pICAST OMC vector.

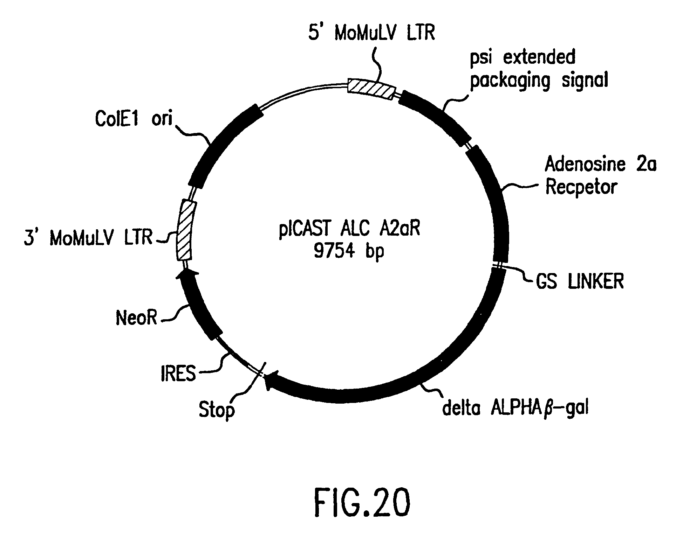

FIGURE 20. pICAST ALC A2aR: Vector for expression of β-galΔα as a C-terminal fusion to Adenosine 2a Receptor. The coding sequence of human Adenosine 2a Receptor (Genebank Accession Number: NM_000675) was cloned in frame to β-galΔα in a pICAST ALC vector.

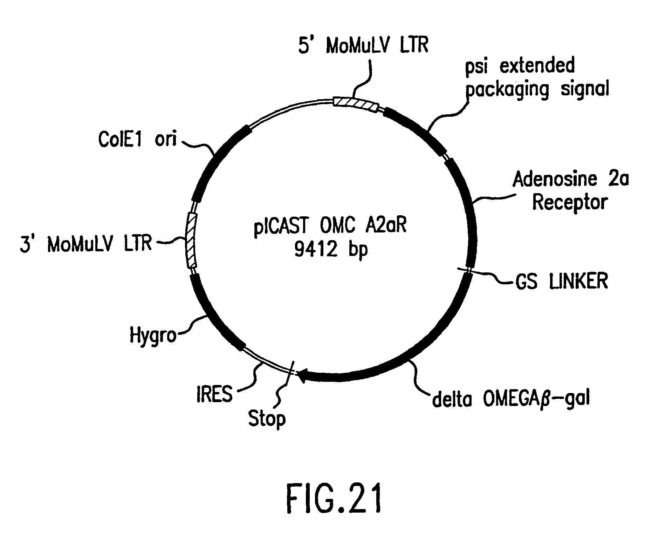

FIGURE 21. pICAST OMC A2aR: Vector for expression of β-galΔω as a C-terminal fusion to Adenosine 2a Receptor. The coding sequence of human Adenosine 2a Receptor (Genebank Accession Number: NM_000675) was cloned in frame to β-galΔω in a pICAST OMC vector.

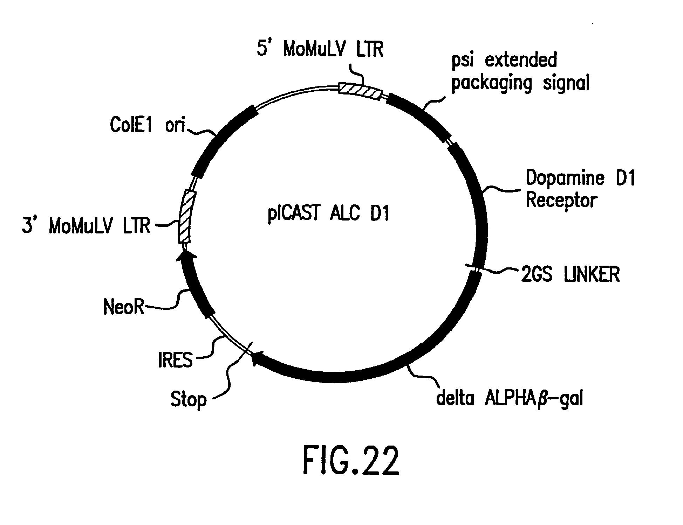

FIGURE 22. pICAST ALC D1: Vector for expression of β-galΔα as a C-terminal fusion to Dopamine D1 Receptor. The coding sequence of human Dopamine D1 Receptor (Genebank Accession Number: X58987) was cloned in frame to β-galΔα in a pICAST ALC vector.
FIGURE 23. A schematic depicting the method of the application, which shows that two inactive mutants that become active when they interact.

### DETAILED DESCRIPTION OF PREFERRED EMBODIMENTS

The present invention provides a method to interrogate GPCR function and pathways. The G-protein-coupled superfamily continues to expand rapidly as new receptors are discovered through automated sequencing of cDNA libraries or genomic DNA. It is estimated that several thousand GPCRs may exist in the human genome, as many as 250 GPCRs have been cloned and only as few as 150 have been associated with ligands. The means by which these, or newly discovered orphan receptors, will be associated with their cognate ligands and physiological functions represents a major challenge to biological and biomedical research. The identification of an orphan receptor generally requires an individualized assay and a guess as to its function. The interrogation of a GPCR's signaling behavior by introducing a replacement receptor eliminates these prerequisites because it can be performed with and without prior knowledge of other signaling events. It is sensitive, rapid and easily performed and should be applicable to nearly all GPCRs because the majority of these receptors should desensitize by a common mechanism.

Various approaches have been used to monitor intracellular activity in response to a stimulant, e.g., enzyme-linked immunosorbent assay (ELISA); Fluorescense Imaging Plate Reader assay (FLIPR^{™}, Molecular Devices Corp., Sunnyvale, CA); EVOscreen^{™}, EVOTEC^{™}, Evotec Biosystems Gmbh, Hamburg, Germany; and techniques developed by CELLOMICS^{™}, Cellomics, Inc., Pittsburgh, PA.

Germino, F.J., et al., "Screening for in vivo protein-protein interactions." Proc. Natl. Acad. Sci., 90(3): 933-7 (1993), discloses an *in vivo* approach for the isolation of proteins interacting with a protein of interest.

Phizicky, E.M., et al., "Protein-protein interactions: methods for detection and analysis." Microbiol. Rev., 59(1): 94-123 (1995), discloses a review of biochemical, molecular biological and genetic methods used to study protein-protein interactions.

Offermanns, et al., "Gα15 and Gα16 Couple a Wide Variety of Receptors to Phospholipase C." J. Biol. Chem., 270(25):15175-80 (1995), discloses that Gα₁₅ and Gα₁₆ can be activated by a wide variety of G-protein-coupled receptors. The selective coupling of an activated receptor to a distinct pattern of G-proteins is regarded as an important requirement to achieve accurate signal transduction. Id.

Barak et al., "A β-arrestin/Green Fluorescent Protein Biosensor for Detecting G Protein-coupled Receptor Activation." J. Biol. Chem., 272(44):27497-500 (1997) and U.S. Patent No. 5,891,646, disclose the use of a β-arrestin/green fluorescent fusion protein (GFP) to monitor protein translocation upon stimulation of GPCR.

The present invention involves a method for monitoring protein-protein interactions in GPCR pathways as a complete assay using ICAST™ (Intercistronic Complementation Analysis Screening Technology as disclosed in pending U.S. patent application serial no. 053,164, filed April 1, 1998). This invention enables an array of assays, including GPCR binding assays, to be achieved directly within the cellular environment in a rapid, non-radioactive assay format amenable to high-throughput screening. Using existing technology, assays of this type are currently performed in a non-cellular environment and require the use of radioisotopes.

The present invention combined with Tropix ICAST^{™} and Advanced Discovery Sciences^{™} technologies, e.g., ultra high-throughput screening, provide highly sensitive cellbased methods for interrogating GPCR pathways which are amendable to high-throughput screening (HTS). These methods are an advancement over the invention disclosed in U.S. Patent 5,891,646, which relies on microscopic imaging of GPCR components as fusion with Green-fluorescent-protein. Imaging techniques are limited by low-throughput, lack of thorough quantification and low signal to noise ratios. Unlike yeast-based-2-hybrid assays used to monitor protein/protein interactions in high-throughput assays, the present invention is applicable to a variety of cells including mammalian cells, plant cells, protozoa cells such as E. coli and cells of invertebrate origin such as yeast, slime mold *(Dictyostelium)* and insects; detects interactions at the site of the receptor target or downstream target proteins rather than in the nucleus; and does not rely on indirect read-outs such as transcriptional activation. The present invention provides assays with greater physiological relevance and fewer false negatives.

Advanced Discovery Sciences^{™} is in the business of offering custom-developed screening assays optimized for individual assay requirements and validated for automation. These assays are designed by HTS experts to deliver superior assay performance. Advanced Discovery Sciences'^{™} custom assay development service encompasses the design, development, optimization and transfer of high performance screening assays. Advanced Discovery Sciences^{™} works to design new assays or convert existing assays to ultra-sensitive luminescent assays ready for the rigors of HTS. Among some of the technologies developed by Advanced Discovery Sciences™ are the cAMP-Screen™ immunoassay system. This system provides ultrasensitive determination of cAMP levels in cell lysates. The cAMP-Screen™ assay utilizes the high-sensitivity chemiluminescent alkaline phosphatase (AP) substrate CSPD^{®} with Sapphire-II™ luminescence enhancer.

### EXAMPLE:

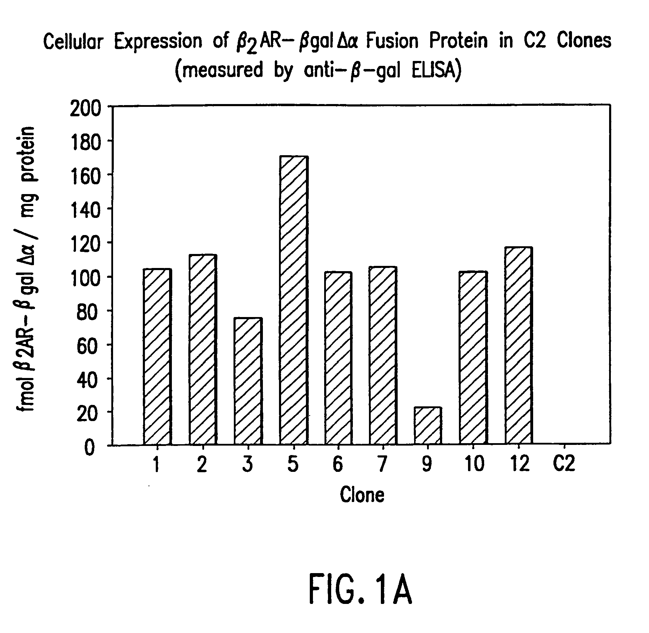

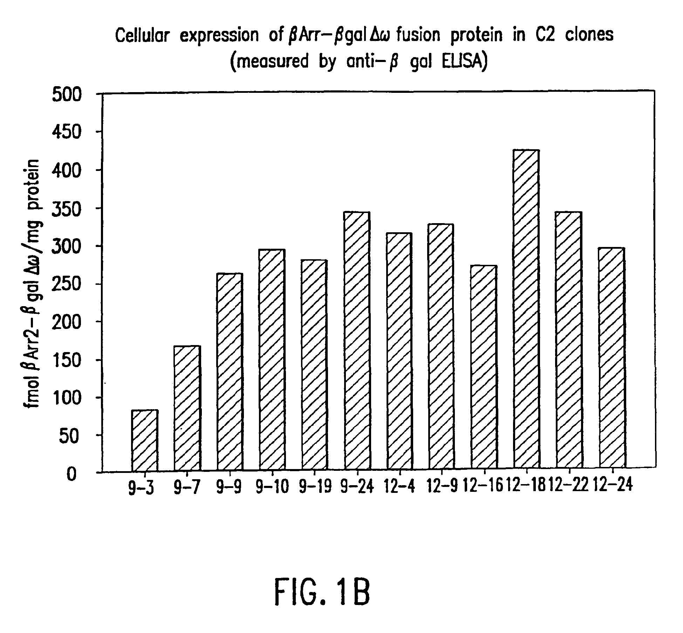

GPCR activation can be measured through monitoring the binding of ligand-activated GPCR by an arrestin. In this assay system, a GPCR, e.g. β adrenergic receptor (β 2AR) and a β arrestin are co-expressed in the same cell as fusion proteins with β gal mutants. As illustrated in Figure 1, the β2AR is expressed as a fusion protein with Δα form of β gal mutant (β2ADRΔα) and the b arrestin as a fusion protein with the Δω mutant of β gal (β-ArrΔω). The two fusion proteins exist inside of a resting (or un-stimulated) cell in separate compartments, i.e. membrane for GPCR and cytosol for arrestin, and they can not form an active b galactosidase enzyme. When such a cell is treated with an agonist or a ligand, the ligand-occupied and activated receptor will become a high affinity binding site for Arrestin. The interaction between an activated β2ADRΔα and β-ArrΔω drives the β gal gal mutant complementation. The enzyme activity can be measured by using an enzyme substrate, which upon cleavage releases a product measurable by colorimetry, fluorescence, chemiluminescence (e.g. Tropix product GalScreen™).

### Experiment protocol-

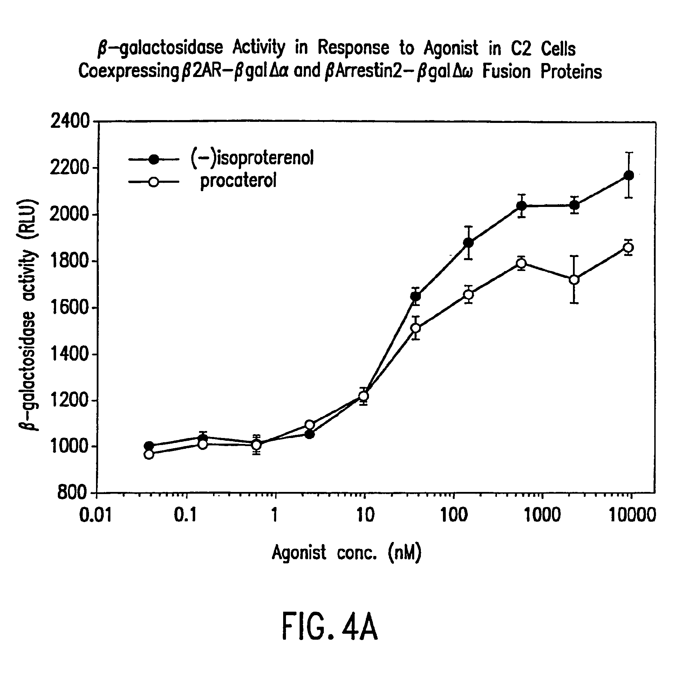

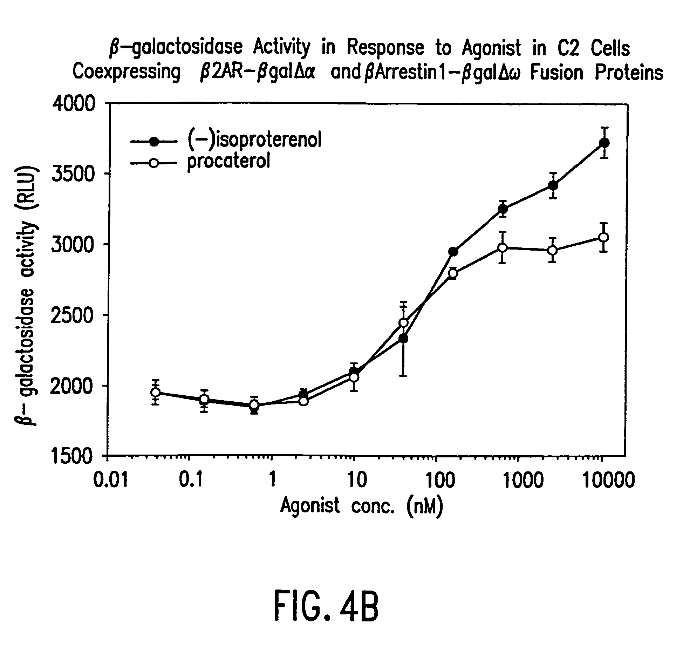

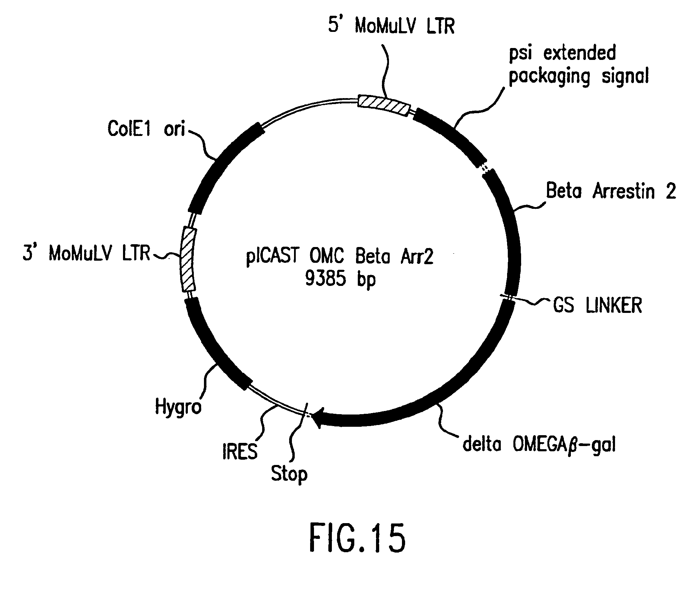

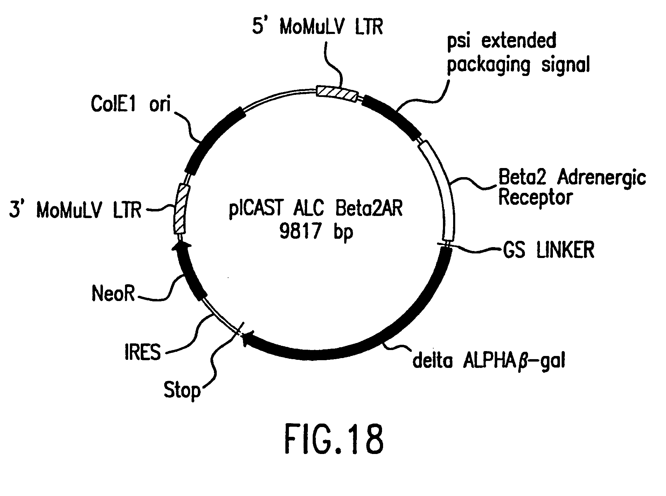

1. In the first step, the expression vectors for β2ADRΔα and βArr2Δω were engineered in selectable retroviral vectors pICAST ALC, as described in Figure 18 and pICAST OMC, as in Figure 15.
2. In the second step, the two expression constructs were transduced into either C2C12 myoblast cells, or other mammalian cell lines, such as COS-7, CHO, A431, HEK 293, and CHW. Following selection with antibiotic drugs, stable clones expressing both fusion proteins at appropriate levels were selected.
3. In the last step, the cells expressing both β2ADRΔα and βArr2Δω were tested for response by agonist/ligand stimulated β galactosidase activity. Triplicate samples of cells were plated at 10,000 cells in 100 microliter volume into a well of 96-well culture plate. Cells were cultured for 24 hours before assay. For agonist assay (Figure 3 and 4), cells were treated with variable concentrations of agonist, for example, (-) isoproterenol, procaterol, dobutamine, terbutiline or L-L-phenylephrine for 60 min at 37C. The induced β galatosidase activity was measured by addition of Tropix GalScreenTM substrate (Applied Biosystems) and luminescence measured in a Tropix TR717TM luminometer (Applied Biosystems). For antagonist assay (Figure 5), cells were pre-incubated for 10 min in fresh medium without serum in the presence of ICI-118,551 or propranolol followed by addition of 10 micro molar (-) isoproterenol.

The assays of this invention, and their application and preparation have been described both generically, and by specific example. The examples are not intended as limiting. Other substituent identities, characteristics and assays will occur to those of ordinary skill in the art, without the exercise of inventive faculty. Such modifications remain within the scope of the invention, unless excluded by the express recitation of the claims advanced below.

## Claims

1. An *ex vivo* method of assessing the effect of a test condition on G-protein-coupled receptor (GPCR) pathway activity, comprising:
a) providing a cell that expresses a GPCR as a fusion protein to a first inactive form of a reporter enzyme and an arrestin as a fusion protein to a second inactive mutant form of the reporter enzyme, wherein interaction of the GPCR and the arrestin results in complementation of the first and second inactive mutant forms of the reporter enzyme thereby forming an active reporter enzyme;
b) exposing the cell to a ligand for said GPCR under said test condition; and
c) detecting enzymatic activity of the reporter enzyme;
wherein increased reporter enzyme activity in the cell compared to that which occurs in the absence of said test condition indicates increased GPCR interaction with the arrestin compared to that which occurs in the absence of said test condition, and decreased reporter enzyme activity in the cell compared to that which occurs in the absence of said test condition indicates decreased GPCR interaction with the arrestin compared to that which occurs in the absence of said test condition; wherein the GPCR and the first mutant form of reporter enzyme are linked together by a polypeptide linker, said linker consisting of the formula - (GGGGS) -; and
wherein the reporter enzyme is β-galactosidase.

2. The method according to claim 1, wherein the test condition is the presence in the cell of a kinase.

3. The method according to claim 1, wherein the test condition is the presence in the cell of a G-protein.

4. The method according to claim 1, wherein the test condition is the exposure of the cell to a compound selected from GPCR agonists and GPCR antagonists.

5. The method according to claim 1, wherein the test condition is co-expression in the cell of a second receptor.

6. The method according to claim 5, wherein the second receptor is a GPCR receptor.

7. The method according to claim 1, wherein the second mutant form of the reporter enzyme is linked to the c-terminal of the arrestin protein.

8. The method according to claim 1, wherein the test condition is the presence of the arrestin as a fusion protein to a second inactive mutant form of the reporter enzyme, wherein the arrestin is a β-arrestin protein
wherein an increase in enzymatic activity in the cell indicates β-arrestin protein binding to the activated GPCR.

9. The method according to claim 8, wherein the second mutant form of the reporter enzyme is linked to the C-terminal of the arrestin protein.

10. The method according to claim 1, wherein the test condition is the presence a test compound, wherein the test compound is added as the ligand, and wherein increased reporter enzyme activity after exposure of the cell to the test compound indicates GPCR agonist activity of the test compound.

11. The method according to claim 10, wherein the cell expresses a GPCR whose function is known.

12. The method according to claim 10, wherein the cell expresses a GPCR whose function is unknown.

13. The method according to claim 10, wherein the cell expresses an odorant or taste GPCR.

14. The method according to claim 10, wherein the cell expresses a β-adrenergic receptor.

15. The method according to claim 10, wherein the cell is selected from the group consisting of mammalian cells, cells of invertebrate origin, plant cells and protozoa cells.

16. The method according to claim 10, wherein the cell endogenously expresses a GPCR.

17. The method according to claim 10, wherein the cell has been transformed to express a GPCR not endogenously expressed by such a cell.

18. The method according to claim 10, wherein the cell expresses a variety of G-protein-coupled receptors.

19. The method according to claim 10, wherein the second mutant form of the reporter enzyme is linked to the C-terminal of the arrestin protein.

20. The method according to claim 1, wherein the test condition is the presence of agonist for the GPCR and the ligand is a test compound, where exposure to the agonist occurs at the same time as, or subsequent to, exposure to the test compound, and wherein decreased reporter enzyme activity after exposure of the cell to the test compound indicates that the test compound is an antagonist for said GPCR.

21. The method according to claim 20, wherein the second mutant form of the reporter enzyme is linked to the C-terminal of the arrestin protein.

22. A substrate having deposited thereon a plurality of cells, said cells expressing at least one GPCR as a fusion protein to a first mutant form of reporter enzyme and an arrestin protein as a fusion to a second mutant form of the reporter enzyme wherein interaction of the GPCR and the arrestin results in complementation of the first and second inactive mutant forms of the reporter enzyme thereby forming an active reporter enzyme, and wherein the GPCR and the first mutant form of reporter enzyme are linked together by a polypeptide linker, said linker consisting of the formula -(GGGGS)ₙ-, and wherein the reporter enzyme is β-galactosidase.

23. The substrate according to claim 22, wherein the substrate contains an enzyme-labile chemical group which, upon cleavage by the reporter enzyme, releases a product measurable by colorimetry, fluorescence or chemiluminescence.

## Patentansprüche

1. Ex-vivo-Verfahren zum Bewerten der Auswirkung einer Testbedingung auf die Aktivität des Weges von G-Protein-gekoppeltem Rezeptor (GPCR), umfassend:
a) Bereitstellen einer Zelle, die einen GPCR als ein Fusionsprotein mit einer ersten inaktiven Form eines Reporterenzyms und ein Arrestin als ein Fusionsprotein mit einer zweiten inaktiven mutanten Form des Reporterenzyms exprimiert, wobei Wechselwirkung des GPCR und des Arrestins in Komplementierung der ersten und zweiten inaktiven mutanten Form des Reporterenzyms resultiert, wodurch ein aktives Reporterenzym erzeugt wird;
b) Einwirken eines Liganden für den GPCR unter der Testbedingung auf die Zelle; und
c) Nachweisen enzymatischer Aktivität des Reporterenzyms;
wobei erhöhte Reporterenzymaktivität in der Zelle, verglichen mit der, welche in Abwesenheit der Testbedingung erfolgt, erhöhte GPCR-Wechselwirkung mit dem Arrestin, verglichen mit der, welche in Anwesenheit der Testbedingung erfolgt, anzeigt und verringerte Reporterenzymaktivität in der Zelle, verglichen mit der, welche in Abwesenheit der Testbedingung erfolgt, verringerte GPCR-Wechselwirkung mit dem Arrestin, verglichen mit der, welche in Abwesenheit der Testbedingung erfolgt, anzeigt; wobei der GPCR. und die erste mutante Form des Reporterenzyms durch einen Polypeptid-Linker miteinander verknüpft werden, wobei der Linker aus der Formel - (GGGGS)ₙ- besteht; und
wobei das Reporterenzym β-Galaclosidase ist.

2. Verfahren gemäß Anspruch 1, wobei die Testbedingung das Vorhandensein einer Kinase in der Zelle ist.

3. Verfahren gemäß Anspruch 1, wobei die Testbedingung das Vorhandensein eines G-Proteins in der Zelle ist.

4. Verfahren gemäß Anspruch 1, wobei die Testbedingung die Einwirkung einer Verbindung, ausgewählt aus GPCR-Agonisten und GPCR-Antagonisten, auf die Zelle ist.

5. Verfahren gemäß Auspruch 1, wobei die Testbedingung die Coexpressien eines zweiten Rezeptors in der Zelle ist.

6. Verfahren gemäß Anspruch 5, wobei der zweite Rezeptor ein GPCR-Rezeptor ist.

7. Verfahren gemäß Anspruch 1, wobei die zweite mutante Form des Reporterenzyms mit dem C-Terminus des Arrestin-Proteins verknüpft wird.

8. Verfahren gemäß Anspruch 1, wobei die Testbedingung das Vorhandensein des Arrestins als eines Fusionsproteins mit einer zweiten inaktiven mutanten Form des Reporterenzyms ist, wobei das Arrestin ein β-Arrestin-Protein ist,
wobei eine Zunahme in enzymatischer Aktivität in der Zelle β-Arrestin-Protein-Bindung an den aktivierten GPCR anzeigt.

9. Verfahren gemäß Anspruch 8, wobei die zweite mutante Form des Reporterenzyms mit dem C-Terminus des Arrestin-Proteins verknüpft wird.

10. Verfahren gemäß Anspruch 1, wobei die Testbedingung das Vorhandensein einer Testverbindung ist, wobei die Testverbindung als der Ligand hinzugegeben wird und wobei erhöhte Reporterenzymaktivität nach Einwirkung der Testverbindung auf die Zelle GPCR-Agonist-Aktivität der Testverbindung anzeigt.

11. Verfahren gemäß Anspruch 10, wobei die Zelle einen GPCR exprimiert, dessen Funktion bekannt ist.

12. Verfahren gemäß Anspruch 10, wobei die Zelle einen GPCR exprimiert, dessen Funktion unbekannt ist.

13. Verfahren gemäß Anspruch 10, wobei die Zelle einen Riechstoff- oder Geschmacks-GPCR exprimiert.

14. Verfahren gemäß Anspruch 10, wobei die Zelle einen β-adrenergen Rezeptor exprimiert.

15. Verfahren gemäß Anspruch 10, wobei die Zelle aus der Gruppe, bestehend aus Säugerzellen, Zellen mit Ursprung von Wirbellosen, Pflanzenzellen und Protozoa-Zellen, ausgewählt ist.

16. Verfahren gemäß Anspruch 10, wobei die Zelle endogen einen GPCR exprimiert.

17. Verfahren gemäß Anspruch 10, wobei die Zelle transformiert worden ist, um einen GPCR, nicht endogen exprimiert durch eine derartige Zelle, zu exprimieren.

18. Verfahren gemäß Anspruch 10, wobei die Zelle eine Vielfalt von G-Proteingekoppelten Rezeptoren exprimiert.

19. Verfahren gemäß Anspruch 10, wobei die zweite mutante Form des Reporterenzyms mit dem C-Terminus des Arrestin-Proteins verknüpft wird.

20. Verfahren gemäß Anspruch 1, wobei die Testbedingung das Vorhandensein von Agonist für den GPCR ist und der Ligand eine Testverbindung ist, wo die Einwirkung des Agonisten zur gleichen Zeit wie oder anschließend an die Einwirkung der Testverbindung erfolgt und wobei verringerte Reporterenzymaktivität nach Einwirkung der Testverbindung auf die Zelle anzeigt, daß die Testverbindung ein Antagonist für den GPCR ist.

21. Verfahren gemäß Anspruch 20, wobei die zweite mutante Form des Reporterenzyms mit dem C-Terminus des Arrestin-Proteins verknüpft wird.

22. Substrat mit einer darauf abgelagerten Vielzahl von Zellen, wobei die Zellen mindestens einen GPCR als ein Fusionsprotein mit einer ersten mutanten Form von Reporterenzym und ein Arrestin-Protein als eine Fusion mit einer zweiten mutanten Form des Reporterenzyms exprimieren, wobei Wechselwirkung des GPCR und des Arrestins in Komplementierung der ersten und zweiten inaktiven mutanten Form des Reporterenzyms resultiert, wodurch ein aktives Reporterenzym erzeugt wird, und wobei der GPCR und die erste mutante Form des Reporterenzyms miteinander durch einen Polypeptid-Linker verknüpft werden, wobei der Linker aus der Formel -(GGGGS)ₙ- besteht und wobei das Reporterenzym β-Galactosidase ist.

23. Substrat gemäß Anspruch 22, wobei das Substrat eine Enzym-labile chemische Gruppe enthält, welche bei Spaltung durch das Reporterenzym ein Produkt, meßbar durch Kalorimetrie, Fluoreszenz oder Chemolumineszenz, freisetzt.

## Revendications

1. Méthode ex vivo d'évaluation de l'effet d'une condition d'essai sur l'activité de la voie de récepteurs couplés aux protéines G (GPCR), comprenant:
a) la fourniture d'une cellule qui exprime un GPCR comme une protéine de fusion avec une première forme inactive d'une enzyme rapporteuse et une arrestine comme une protéine de fusion avec une deuxième forme mutante inactive de l'enzyme, rapporteuse, dans laquelle l'interaction du GPCR et de l'arrestine conduit à une complémentation des première et deuxième formes mutantes inactives de l'enzyme rapporteuse formant ainsi une enzyme rapporteuse active;
b) l'exposition de la cellule à un ligand pour ledit GPCR dans ladite condition d'essai; et
c) la détection de l'activité enzymatique de l'enzyme rapporteuse;
dans laquelle l'activité accrue de l'enzyme rapporteuse dans la cellule comparée à celle qui se produit en l'absence de ladite condition d'essai indique une interaction accrue de GPCR avec l'arrestine comparée à celle qui se produit en l'absence de ladite condition d'essai, et l'activité diminuée de l'enzyme rapporteuse dans la cellule comparée à celle qui se produit en l'absence de ladite condition d'essai indique une interaction diminuée de GPCR avec l'arrestine comparée à celle qui se produit en l'absence de ladite condition d'essai; dans laquelle le GPCR et la première forme mutante de l'enzyme rapporteuse sont liés ensemble par un lieur polypeptidique, ledit lieur étant constitué de la formule - (GGGGS)ₙ-; et
dans laquelle l'enzyme rapporteuse est la β-galactosidase.

2. Méthode selon la revendication 1, dans laquelle la condition d'essai est la présence dans la cellule d'une kinase.

3. Méthode selon la revendication 1, dans laquelle la condition d'essai est la présence dans la cellule d'une protéine G.

4. Méthode selon la revendication 1, dans laquelle la condition d'essai est l'exposition de la cellule à un composé choisi parmi des agonistes de GPCR et des antagonistes de GPCR.

5. Méthode selon la revendication 1, dans laquelle la condition d'essai est une co-expression dans la cellule d'un deuxième récepteur.

6. Méthode selon la revendication 5, dans laquelle le deuxième récepteur est un récepteur GPCR.

7. Méthode selon la revendication 1, dans laquelle la deuxième forme mutante de l'enzyme rapporteuse est liée à l'extrémité C-terminale de la protéine d'arrestine.

8. Méthode selon la revendication 1, dans laquelle la condition d'essai est la présence de l'arrestine comme une protéine de fusion avec une deuxième forme mutante inactive de l'enzyme rapporteuse, dans laquelle l'arrestine et une protéine β-arrestine,
dans laquelle une augmentation dans l'activité enzymatique dans la cellule indique la liaison de la protéine β-arrestine avec le GPCR active.

9. Méthode selon la revendication 8, dans laquelle la deuxième forme mutante de l'enzyme rapporteuse est liée à l'extrémité C-terminale de la protéine d'arrestine.

10. Méthode selon la revendication 1, dans laquelle la condition d'essai est la présence d'un composé d'essai, dans laquelle le composé d'essai est ajouté en tant que ligand et dans laquelle l'activité accrue de l'enzyme rapporteuse après l'exposition de la cellule au composé d'essai indique l'activité d'agoniste de GPCR du composé d'essai.

11. Méthode selon la revendication 10, dans laquelle la cellule exprime un GPCR dont la fonction est connue.

12. Méthode selon la revendication 10, dans laquelle la cellule exprime un GPCR dont la fonction est inconnue.

13. Méthode selon la revendication 10, dans laquelle la cellule exprime un GPCR odorant ou de goût.

14. Méthode selon la revendication 10, dans laquelle la cellule exprime un récepteur β-adrénergique.

15. Méthode selon la revendication 10, dans laquelle la cellule est choisie dans le groupe constitué de cellules mammifères, de cellules d'origine invertébrée, de cellules végétales et de cellules de protozoaires.

16. Méthode selon la revendication 10, dans laquelle la cellule exprime de façon endogène un GPCR.

17. Méthode selon la revendication 10, dans laquelle la cellule a été transformée pour exprimer un GPCR exprimé de façon non endogène par une telle cellule.

18. Méthode selon la revendication 10, dans laquelle la cellule exprime une variété de récepteurs couplés aux protéines G.

19. Méthode selon la revendication 10, dans laquelle la deuxième forme mutante de l'enzyme rapporteuse est liée à l'extrémité C-terminale de la protéine d'arrestine.

20. Méthode selon la revendication 1, dans laquelle la condition d'essai est la présence d'un agoniste pour le GPCR et le ligand est un composé d'essai, où l'exposition à l'agoniste se produit au même moment que, ou à la suite de, l'exposition au composé d'essai, et dans laquelle l'activité diminuée de l'enzyme rapporteuse après l'exposition de la cellule au composé d'essai indique que le composé d'essai est un antagoniste pour ledit GPCR.

21. Méthode selon la revendication 20, dans laquelle la deuxième forme mutante de l'enzyme rapporteuse est liée à l'extrémité C-terminale de la protéine d'arrestine

22. Substrat possédant, exposée sur celui-ci, une pluralité de cellules, lesdites cellules exprimant au moins un GPCR comme une protéine de fusion avec la première forme mutante de l'enzyme rapporteuse et une protéine d'arrestine comme une fusion avec une deuxième forme mutante de l'enzyme rapporteuse, dans lequel l'interaction du GPCR et de l'arrestine conduit à une complémentation des première et deuxième formes mutantes inactives de l'enzyme rapporteuse formant ainsi une enzyme rapporteuse active et dans lequel le GPCR et la première forme mutante de l'enzyme rapporteuse sont liés ensemble par un lieur polypeptidique, ledit lieur étant constitué de la formule -(GGGGS)ₙ- et dans lequel l'enzyme rapporteuse est une β-galactosidase.

23. Substrat selon la revendication 22, où le substrat contient un groupe chimique labile par une enzyme qui, à la suite d'un clivage par l'enzyme rapporteuse, libère un produit mesurable par colorimétrie, fluorescence ou chimiluminescence.
